(19)

**Europäisches Patentamt**
**European Patent Office**
**Office européen des brevets**

(11) **EP 4 588 999 A1**

(12) **EUROPEAN PATENT APPLICATION**

(43) Date of publication:
**23.07.2025 Bulletin 2025/30**

(21) Application number: **24152625.0**

(22) Date of filing: **18.01.2024**

(51) International Patent Classification (IPC):
***C12M 3/00*** *(2006.01)*     ***C12M 1/12*** *(2006.01)*
***C12M 3/06*** *(2006.01)*

(52) Cooperative Patent Classification (CPC):
**C12M 21/08; C12M 23/44; C12M 25/10; C12M 27/14**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Cellular Agriculture Ltd**
**Llanelli Carmarthenshire SA15 5TL (GB)**

(72) Inventors:
• **ARGYLE, Iain**
**Llanelli, SA15 5TL (GB)**

• **ELLIS, Marianne**
**Llanelli, SA15 5TL (GB)**
• **ALLAN, Scott**
**Llanelli, SA15 5TL (GB)**
• **DUNSFORD, Illtud**
**Llanelli, SA15 5TL (GB)**
• **SOMERVILLE-ROBERTS, Nigel**
**Llanelli, SA15 5TL (GB)**

(74) Representative: **HGF**
**HGF Limited**
**1 City Walk**
**Leeds LS11 9DX (GB)**

(54) **A MEAT CULTURE CONSTRUCT FOR A MEAT CULTURE BIOREACTOR MODULE**

(57)    A meat culture construct for a meat culture bioreactor module (102) that is attachable to a meat culturing system (100). The meat culture bioreactor module (102) has a housing (108) adapted to receive the meat culture construct for culturing cells on the meat culture construct within the housing. The meat culture construct comprises a plurality of fibres (120) and an end support (126). The plurality of fibres (120) have external surfaces for cell adhesion and culturing, and ends embedded within the end support (126) and extending from the end support (126) such they extend into the housing (108) when the meat culture construct is received in the housing of the meat culture bioreactor module (102). At a plane coincident with the end support (126) the plurality of fibres (120) are arranged in a fibre distribution defining an envelope. The fibre distribution comprises a Fibre Distribution Parameter, FDP, of between 9 cm$^3$ and 24 cm$^3$, the FDP being the product of the sum of the circumferences of the plurality of fibres within the envelope and the area within the envelope not occupied by the plurality of fibres.

**EP 4 588 999 A1**

**Description**

## FIELD OF THE INVENTION

The present invention relates to a meat culture construct for a meat culture bioreactor module, a meat culture bioreactor module, a meat culturing system, a method of seeding muscle cells or muscle cell precursors in a meat culture bioreactor module, and a method of culturing meat.

## BACKGROUND

Production of cultured meat by the in-vitro culturing of cells of animal origin, such as muscle cells, muscle precursor cells and fat cells, is of increasing interest for food production. Lab-scale production of cultured meat in its simplest form of muscle cells or cocultures of muscle and fat cells has been achieved, however scaling-up the process to make a viable economic product is still challenging.

It is known to use hollow fibre bioreactors for culturing cells by perfusion, where cell culture media is provided through the lumina of the hollow fibres to feed cells growing on external surfaces of the hollow fibres. Typically, such bioreactors are designed to maximise the number of fibres within the bioreactor by making the fibres as small as possible and by providing a high density of fibres within the bioreactor module. This increases the surface area for growing cells and for nutrient exchange, and so increases the production of cells. Accordingly, bioreactors are typically constructed with small diameter fibres packed at high density.

Due to the large-scale requirements for commercial meat production, it is necessary to improve aspects of cell culturing, as well as the designs of any bioreactor systems used for such culturing.

In particular, there is a need to provide an improved bioreactor that is designed for efficient seeding of muscle cells onto a construct, and which also provides for improved meat culturing, harvesting and collection. There is also a need for the bioreactor to be robust for reliable and economic production of cultured meat at scale.

## SUMMARY OF THE INVENTION

There are competing parameters when designing a fibre bioreactor for cultured meat production - the fibre surface area for the cells to adhere to, the space for the cultured meat (muscle cells) to grow (taking into account how those cells will be supplied with nutrients), and the robustness of the fibres for the meat culturing process, including during cell growth and harvesting. The inventors have advantageously defined a meat culture construct and meat culture bioreactor module that balances these parameters and provides for improved meat culturing.

According to the present invention there is provided a meat culture construct for a meat culture bioreactor module that is attachable to a meat culturing system. The meat culture bioreactor module has a housing adapted to receive the meat culture construct for culturing cells on the meat culture construct within the housing.

The meat culture construct comprises a plurality of fibres and an end support. The plurality of fibres having external surfaces for cell adhesion and culturing, and have ends embedded within the end support and extend from the end support such they extend into the housing when the meat culture construct is received in the housing of the meat culture bioreactor module.

At a plane coincident with the end support the plurality of fibres are arranged in a fibre distribution defining an envelope.

### Fibre Distribution Parameter (FDP)

In examples, the fibre distribution comprises a Fibre Distribution Parameter, FDP, of between 9 $cm^3$ and 24 $cm^3$. The FDP is the product of the sum of the circumferences of the plurality of fibres within the envelope and the area within the envelope not occupied by the plurality of fibres.

In other words, the FDP is the product of the seeding length (SL - sum of the circumferences of the plurality of fibres) and the growing area (GA - area within the envelope not occupied by the plurality of fibres) in the two dimensional plane at the end support of the meat culture construct.

The cells to be cultured, in particular muscle cells or muscle cell precursors, are typically introduced into the construct as a suspension of individual cells and need to adhere, via electrostatic forces and then attachment proteins, to external surfaces of the plurality of fibres. Once adhered to the external surfaces of the fibres the cells can be cultured (i.e., the cells can grow) to form cultured meat. This adhesion step is known as "seeding". The seeding step is important for efficient cultured meat production. If seeding is not efficient then subsequent steps will inherently deliver a sub-optimal outcome.

Within the plane, the seeding length provided by the plurality of fibres is the sum of the circumferences of the plurality of fibres at the end support. The total seeding area is the seeding length multiplied by the lengths of the fibres.

During seeding, a cell suspension (for example with muscle cells or muscle cell precursors) introduced into the cavity can circulate about the plurality of fibres within the cavity due to gravity and/fluid flow, and seed to the external surfaces of the plurality of fibres. It is advantageous if as many of the introduced cells as possible become adhered to an external surface of the plurality of fibres. Advantageously, as described below, the defined ranges of FDP provide space within the cavity to allow the cells to reach substantially all of the plurality of fibres and to substantially evenly seed (adhere) to all of the outer surfaces of the plurality of fibres. Subsequently, the seeded cells can be cultured to form cultured meat, and the cultured meat may thereby be formed substantially evenly on the external surfaces of the plurality of fibres. The defined range of FDP also provides space for the cells to grow and form cultured meat in an efficient manner.

After seeding the cells are cultured in a cell growth process. During the cell growth process a cell culture media, for example containing nutrients (including e.g., oxygen) to encourage cell growth, may be provided in, or circulated through, the housing (via the fibres or directly in the housing). The cell culturing process may be a perfusion process. During the cell growth process the cells grow to form cultured meat on the external surfaces of the fibres, and therefore grow into the spaces between the fibres - the growing area. Specifically, the growing area is the space within the meat culture construct that is not occupied by the fibres and so provides space for the cells to grow into.

The seeding length and the growing area counteract each other. For a given fibre diameter, a large seeding length (more fibres) reduces the growing area, and a large growing area (fewer fibres) reduces the seeding length. In particular, if the seeding length is increased by providing a higher fibre density then the surface area of the fibres is increased but the spaces between the fibres are reduced, inhibiting circulation of cells during seeding and reducing the available space for meat to culture. On the other hand, if a lower fibre density is provided then seeding is more efficient (cells can reach more of the fibre surfaces), but there is less surface area on which cells can seed, reducing the amount of meat that can be cultured. There are other factors that also influence the efficiency and effectiveness of the meat culture construct, as discussed below.

The inventors have improved known meat culture constructs by balancing the seeding length and the growing area and by taking account of other factors as set out below, and defining an advantageous FDP range of 9 cm$^3$ to 24 cm$^3$. This is in contrast to a conventional bioreactor designs in which the fibre surface area is maximised by providing smaller and more numerous fibres.

The seeding length (SL) is the product of the number of fibres (N) within a unit area, and the external surfaces (circumferences) of the fibres having a diameter (d):

$$SL = N \times Pi \times d$$

The growing area (GA) is the area within the unit area that is not occupied by the fibres (i.e., the free volume). The growing area (GA) does not take account of whether or not the cells are able to grow into that area, it is just the volume within the envelope not occupied by the fibres themselves. The growing area is therefore the unit area (1 in this example) minus the area occupied by the fibres with diameter (d), and can be written as:

$$GA = 1 - (N \times Pi \times (0.5d)^2)$$

By re-writing the seeding length equation for N, the growing area (GA) can be written as:

$$GA = 1 - (SL \times d) / 4$$

It can be seen that the seeding length and the growing area are both functions of the diameter (d) of the fibres and the number of fibres (N) provided per unit area (fibre density). It is therefore advantageous to select the fibre diameter and fibre density to provide improved seeding and culturing of cells for meat culturing. The FDP, being the product of the seeding length (SL) and the growing area (GA), is indicative of the balance between seeding length and growing area, and thus provides a useful parameter. The FDP is defined as:

$$FDP = SL \times GA$$

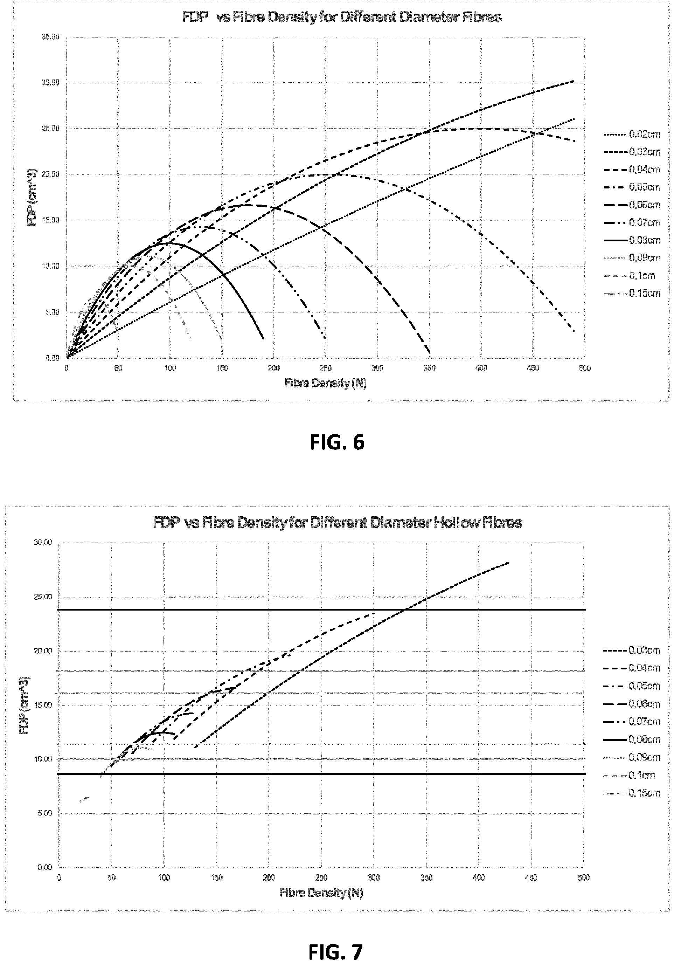

FIG. 6 shows the FDP of various fibre diameters plotted against fibre density (N). As shown in FIG. 6, for each fibre diameter the FDP includes a peak that is indicative of an optimum fibre density that achieves a balance between the seeding length and the growing area. That is, the peak of the FDP for a given fibre diameter (d) indicates the optimum number of fibres (N) per unit area that maximises the seeding length and the growing area. Note that the peak for the 0.3mm diameter fibre is beyond the range of fibre density illustrated (the peak for 0.3mm diameter fibres is at about 700 fibres / cm$^2$).

Based on the information shown in FIG. 6, the advantageous range of FDP is from about 9 cm$^3$ to about 24 cm$^3$. More preferable ranges of the FDP are from about 10 cm$^3$ to about 18 cm$^3$, and from about 11 cm$^3$ to about 16 cm$^3$. These ranges are illustrated by the horizontal lines shown in FIG. 6.

However, the inventors have found that in addition to the seeding length and the growing area it is advantageous to also consider other factors influencing the culturing of meat, which may differ for hollow fibres and solid fibres as described below.

*Fibre Distribution Parameter (FDP) - Hollow Fibres*

In examples, the plurality of fibres comprise hollow fibres. The hollow fibres may be porous.

In such examples, the meat culture bioreactor module, in particular the housing, may comprise a fluid inlet arranged to fluidly connect with the meat culturing system for passing a cell culture media through the lumina of the hollow fibres. Nutrients from the cell culture media diffuse through the walls (specifically through the pores) of the hollow fibres and feed cells growing on the outer surfaces of the hollow fibres.

In examples in which the meat culture bioreactor module comprises a second end support, the bioreactor module may also include an outlet arranged to fluidly connect the hollow fibres at the second end support to the meat culturing system for receiving a cell culture media passed through the hollow fibres.

In alternative examples, cell culture media may be circulated through the cavity to feed nutrients to the cells on the outer surfaces of the hollow fibres. Exudate may be extracted through the lumina of the hollow fibres.

With hollow fibres, an important factor to consider is the thickness of the layer of cultured meat cells on the external surfaces of the fibres the amount of cultured meat that can be generated on any of the fibres. This is limited by the maximum distance that nutrients can effectively travel (e.g., diffuse) through the layers of cells on the external surface of a fibre. This distance is referred to as the Maximum Diffusion Distance, MDD. The MDD is based on nutrient diffusion through the cells, so is independent of the dimensions of the fibres.

It is highly preferable for the structure and texture of the cultured meat if the cultured meat can form as a coherent structure on the meat culture construct. This is best achieved if the cultured cells form one coherent mass of meat in the volume between the fibres of the meat culture construct. This can be achieved by limiting the spacings between the fibres to two times the MDD. In one example, the distance between the fibres (the fibre spacing) is equal to two times the MDD, in which example the cells can grow to occupy the entire growing area. Distances greater than two times the MDD may create regions within the meat culture construct where cells cannot grow because they cannot be supplied with nutrients. The maximum MDD for cultured meat cells is about 300 microns, more preferably about 250 microns more preferably about 200 microns. In addition, there needs to be space for the cells to grow into to provide a cultured meat product, and that space is preferably at least 100 microns. Hence, the fibre spacings within the meat culture construct are preferably between 200 and 600 microns, more preferably between 200 and 500 microns, more preferably between 200 and 400 microns. The inventors have found that this fibre spacing provides adequate space for cells to circulate and adhere to the external surfaces of the fibres during seeding, while reducing the area of the meat culture construct that cannot be supplied with nutrients.

Table 1 shows the maximum fibre density that can be achieved when fibres are packed as efficiently as possible and separated by two times the MDD (where the MDD is 100 microns, 200 microns, 250 microns, and 300 microns). This has been calculated by modelling the maximum packing of fibres having a diameter plus two times the MDD and packed in a triangular pattern. The maximum packing of fibres can be regarded as the maxim packing of circles of uniform diameter into a given area (1 cm x 1 cm). The MDD therefore provides the maximum fibre densities for a given fibre diameter.

*Table 1 - Maximum Number of Fibres per square centimetre by hollow fibre diameter and MDD*

| | **Hollow Fibre Diameter (mm)** | | | | | | | | | |
| **MDD (mm)** | **0.2** | **0.3** | **0.4** | **0.5** | **0.6** | **0.7** | **0.8** | **0.9** | **1.0** | **1.5** |
|---|---|---|---|---|---|---|---|---|---|---|
| **0.1** | 672 | 429 | 304 | 216 | 168 | 126 | 105 | 85 | 68 | 30 |
| **0.2** | 304 | 216 | 168 | 126 | 105 | 85 | 68 | 56 | 52 | 23 |
| **0.25** | 216 | 168 | 126 | 105 | 85 | 68 | 56 | 52 | 42 | 23 |
| **0.3** | 168 | 126 | 105 | 85 | 68 | 56 | 52 | 42 | 39 | 20 |

Another factor to consider is that there is a practical lower limit on the fibre diameter because the cells (muscle cells, muscle precursor cells, fat cells) have to be able to adhere to the outer surfaces of the fibres. If the fibres are too small the cells will not be able to adhere to the outer surfaces of the fibres because the curvature is too great. The inventors have found that muscle cells can adhere to fibres having an outer diameter of 0.3mm, but below this diameter the adhesion rate decreases. For cell adhesion, the fibre diameter is preferably 0.3mm or greater, more preferably 0.5mm or greater.

A further factor is the robustness of the fibres. The inventors have found that fibres with a small diameter (e.g., less than 0.3mm) are not sufficiently stiff or strong for meat culturing applications. In particular, as the meat culture grows it will push, pull, stretch, and compress the fibres in different ways depending on local cell growth, and fibres with a diameter of more than 0.3mm, preferably more than 0.5mm, are beneficially robust enough to withstand such forces. In addition, fibres with a diameter of less than 0.3mm are susceptible to electrostatic forces during handling and assembly into a construct, making them bunch together. This makes them unsuitable for meat culturing applications. Larger diameter fibres have preferred performance with respect to handling and robustness. The inventors have found that fibres with a diameter of 0.3 mm (or 0.5mm) or greater are sufficiently robust for meat culturing.

A further factor for a hollow fibre meat culture construct is the pressure drop within the hollow fibres. In use, a cell culture media may be circulated through the lumina of the hollow fibres to foster cell growth (or exudate is extracted through the hollow fibres), and small fibres (e.g., less than 0.3mm or less than 0.5mm) may have a pressure drop that is too high for effective circulation over the length of the fibres, which may reduce or prevent cell culture media reaching some parts of the meat culture construct, and may cause blockages or ruptures or leaks.

Accordingly, the inventors have realised that the preferred range of the FDP for hollow fibres should also be informed by the MDD, and the minimum diameter of the fibres for cell adhesion, robustness, and pressure drop.

Applying a minimum fibre diameter of 0.3mm, and the maximum fibre densities of the fibres as informed by the MDD, the FDP range broadly corresponds to the preferred range - between 9 cm$^3$ and 24 cm$^3$. Accordingly, the meat culture construct with a FDP of between 9 cm$^3$ and 24 cm$^3$ provides advantages in terms of the balance between seeding length and growing area, and also accounts for the MDD, fibre surface curvature for cell adhesion, the robustness of the fibres, and pressure drop of the hollow fibres.

This is illustrated in FIG. 7 and Table 2. Fig. 7 shows the FDP for hollow fibres having a diameter greater than 0.3mm with the curves truncated within the minimum and maximum fibre densities shown in Table 1 above (minimum fibre density = 0.1mm MDD, maximum fibre density = 0.3mm MDD). The preferred ranges of FDP are indicated by the horizontal lines.

Table 2 provides the data on which the graph of FIG. 7 is based, showing the fibre densities for each diameter of fibre with the FDP. This provides preferred values and ranges of fibre density for each diameter of fibre. In Table 2, the lighter grey highlighted FDP values indicate the preferable range of 9 cm$^3$ to 24 cm$^3$. The darker grey highlighted FDP values indicate more preferred ranges. It can be seen that the FDP and fibre densities for 1.5mm fibres falls outside of the preferred range - these fibres are too large to provide the balance between seeding length and growing area. Accordingly, it can be seen that the preferred range of fibre diameters for the meat culture construct is 0.3 mm to 1 mm, more preferably 0.3 mm to 0.9 mm, more preferably 0.3 mm to 0.8 mm, more preferably 0.4 mm to 0.8mm, more preferably 0.5 mm to 0.8 mm, more preferably 0.5 mm to 0.7 mm.

| Fibre Density (N) | Fibre Diameter | | | | | | | | |
|---|---|---|---|---|---|---|---|---|---|
| | 0.3mm | 0.4mm | 0.5mm | 0.6mm | 0.7mm | 0.8mm | 0.9mm | 1mm | 1.5mm |
| 1 | | | | | | | | | |
| 10 | | | | | | | | | |
| 20 | | | | | | | | | 6.09 |
| 30 | | | | | | | | | 6.64 |
| 40 | | | | | | | 8.43 | 8.62 | |
| 50 | | | | | | 9.41 | 9.64 | 9.54 | |
| 60 | | | | | 10.15 | 10.53 | 10.49 | 9.97 | |
| 70 | | | | 10.58 | 11.25 | 11.40 | 10.98 | 9.90 | |
| 80 | | | | 11.67 | 12.18 | 12.02 | 11.11 | | |
| 90 | | | 11.64 | 12.65 | 12.94 | 12.39 | 10.88 | | |
| 100 | | | 12.62 | 13.52 | 13.53 | 12.50 | | | |
| 110 | | 11.91 | 13.55 | 14.29 | 13.95 | 12.36 | | | |
| 120 | | 12.81 | 14.41 | 14.94 | 14.20 | | | | |
| 130 | 11.13 | 13.67 | 15.21 | 15.50 | 14.29 | | | | |
| 140 | 11.89 | 14.50 | 15.95 | 15.94 | | | | | |
| 150 | 12.64 | 15.30 | 16.62 | 16.28 | | | | | |
| 160 | 13.37 | 16.06 | 17.24 | 16.52 | | | | | |
| 170 | 14.10 | 16.80 | 17.79 | 16.64 | | | | | |
| 180 | 14.81 | 17.50 | 18.28 | | | | | | |
| 190 | 15.50 | 18.18 | 18.71 | | | | | | |
| 200 | 16.18 | 18.82 | 19.08 | | | | | | |
| 210 | 16.85 | 19.43 | 19.39 | | | | | | |
| 220 | 17.51 | 20.00 | 19.63 | | | | | | |
| 230 | 18.15 | 20.55 | | | | | | | |
| 240 | 18.78 | 21.06 | | | | | | | |
| 250 | 19.40 | 21.55 | | | | | | | |
| 260 | 20.00 | 22.00 | | | | | | | |
| 270 | 20.59 | 22.42 | | | | | | | |
| 280 | 21.17 | 22.81 | | | | | | | |
| 290 | 21.73 | 23.16 | | | | | | | |
| 300 | 22.28 | 23.49 | | | | | | | |
| 310 | 22.81 | | | | | | | | |
| 320 | 23.34 | | | | | | | | |
| 330 | 23.85 | | | | | | | | |
| 340 | 24.34 | | | | | | | | |
| 350 | 24.83 | | | | | | | | |
| 360 | 25.30 | | | | | | | | |
| 370 | 25.75 | | | | | | | | |
| 380 | 26.19 | | | | | | | | |
| 390 | 26.62 | | | | | | | | |
| 400 | 27.04 | | | | | | | | |
| 410 | 27.44 | | | | | | | | |
| 420 | 27.83 | | | | | | | | |
| 430 | 28.21 | | | | | | | | |

*Table 2 – FDP by hollow fibre diameter and fibre density (N = fibre density)*

As shown in FIG. 7 and Table 2, in examples, the FDP is preferably between 10 cm$^3$ and 18 cm$^3$. In particular, the fibre diameter may be between 0.5mm and 0.8mm, with the FDP being between 10 cm$^3$ and 18 cm$^3$. This narrower range of FDP provides advantages in terms of the balance between seeding length and growing area, as well as robust fibres that can be packed at an efficient density based on MDD. Accordingly, a meat culture construct having hollow fibres with a fibre distribution with a FDP of between 10 cm$^3$ and 18 cm$^3$ provides improved meat culturing in contrast to known fibre bioreactors that are generally optimised by reducing fibre diameter and increasing the number of fibres (fibre density).

In examples, the FDP is preferably between 11 cm$^3$ and 16 cm$^3$. In particular, the fibre diameter may be between 0.5mm and 0.7mm, the FDP being between 11 cm$^3$ and 16 cm$^3$. This narrower range of FDP provides advantages in terms of the balance between seeding length and growing area, as well as robust fibres that can be packed at an efficient density based on MDD. Accordingly, a meat culture construct having a fibre distribution with a FDP of between 11 cm$^3$ and 16 cm$^3$ provides improved meat culturing in contrast to known fibre bioreactors that are generally optimised by reducing fibre diameter and increasing the number of fibres (fibre density).

In examples, the fibre diameter of the plurality of fibres is at least 0.3mm. In examples, the outer fibre diameter of the plurality of fibres is between 0.3mm and 1 mm, for example between 0.4mm and 0.9mm, for example between 0.5mm and 0.8mm, for example one of 0.3mm, 0.4mm, 0.5mm, 0.6mm, 0.7mm, 0.8mm, 0.9mm or 1mm.

In examples, some of the plurality of fibres may have different outer diameters to others. Fibres with different outer diameters may be distributed evenly through the envelope, or concentrated in one or more locations within the envelope, for example larger fibres may be concentrated towards a centre of the envelope.

In specific examples, the fibres have a diameter of 0.3mm and the meat culture construct has a FDP of between 11 cm$^3$ and 24 cm$^3$, more preferably between 11 cm$^3$ and 18 cm$^3$, more preferably between 11 cm$^3$ and 16 cm$^3$.

In specific examples, the fibres have a diameter of 0.4mm and the meat culture construct has a FDP of between 11 cm$^3$ and 24 cm$^3$, more preferably between 11 cm$^3$ and 18 cm$^3$, more preferably between 11 cm$^3$ and 16 cm$^3$.

In specific examples, the fibres have a diameter of 0.5mm and the meat culture construct has a FDP of between 11 cm$^3$ and 20 cm$^3$, more preferably between 11 cm$^3$ and 18 cm$^3$, more preferably between 11 cm$^3$ and 16 cm$^3$.

In specific examples, the fibres have a diameter of 0.6mm and the meat culture construct has a FDP of between 10 cm$^3$ and 15 cm$^3$, more preferably between 11 cm$^3$ and 15 cm$^3$.

In specific examples, the fibres have a diameter of 0.7mm and the meat culture construct has a FDP of between 10 cm$^3$ and 15 cm$^3$, more preferably between 11 cm$^3$ and 15 cm$^3$.

In specific examples, the fibres have a diameter of 0.8mm and the meat culture construct has a FDP of between 9 cm$^3$ and 13 cm$^3$, more preferably between 10 cm$^3$ and 13 cm$^3$, more preferably between 11 cm$^3$ and 13 cm$^3$.

In specific examples, the fibres have a diameter of 0.9mm and the meat culture construct has a FDP of between 9 cm$^3$ and 11 cm$^3$, more preferably between 10 cm$^3$ and 11 cm$^3$, more preferably about 11 cm$^3$.

In specific examples, the fibres have a diameter of 1mm and the meat culture construct has a FDP of between 9 cm$^3$ and 10 cm$^3$, more preferably about 9 cm$^3$.

*Fibre Density - Hollow Fibres*

It will be appreciated that the advantageous ranges of FDP can be used to define advantageous ranges of fibre density - the number of fibres per unit area. However, the FDP advantageously defines the characteristics of the fibre distribution irrespective of fibre diameter, and so has advantages over defining the fibre density itself, which varies more based on fibre diameter.

Nevertheless, according to another aspect the present invention may be characterised in that the fibre density of the fibre distribution is between about 50 fibres/cm$^2$ and about 330 fibres/cm$^2$. In examples, the fibre density is between about 60 fibres/cm$^2$ and about 220 fibres/cm$^2$, for example between about 70 fibres/cm$^2$ and about 190 fibres/cm$^2$. This is shown in Table 2.

In an example, the fibre diameter is between 0.3mm and 1mm and the fibre density is preferably between about 50 fibres/cm$^2$ and about 330 fibres/cm$^2$, more preferably between about 60 fibres/cm$^2$ and about 220 fibres/cm$^2$, for example between about 70 fibres/cm$^2$ and about 190 fibres/cm$^2$.

In another example the fibre diameter is between 0.5mm and 0.8mm and the fibre density is between about 50 fibres/cm$^2$ and about 220 fibres/cm$^2$, more preferably between about 60 fibres/cm$^2$ and about 170 fibres/cm$^2$, more preferably between about 70 fiibres/cm$^2$ and about 140 fibres/cm$^2$.

In another example, the fibre diameter is between 0.5mm and 0.7mm and the fibre density is between about 60 fibres/cm$^2$ and about 220 fibres/cm$^2$, more preferably between about 60 fibres/cm$^2$ and about 170 fibres/cm$^2$, more preferably between about 70 fiibres/cm$^2$ and about 140 fibres/cm$^2$.

In examples, the fibre diameter is 1mm and the fibre density is between about 50 fibres/cm$^2$ and about 70 fibres/cm$^2$.

In examples, the fibre diameter is 0.3mm and the fibre density is between about 130 fibres/cm$^2$ and about 330 fibres/cm$^2$, more preferably between about 130 fibres/cm$^2$ and about 220 fibres/cm$^2$, more preferably between about 130 fibres/cm$^2$ and about 190 fibres/cm$^2$.

In examples, the fibre diameter is 0.4mm and the fibre density is between about 110 fibres/cm$^2$ and about 300 fibres/cm$^2$, more preferably between about 110 fibres/cm$^2$ and about 180 fibres/cm$^2$, more preferably between about 110 fibres/cm$^2$ and about 150 fibres/cm$^2$.

In examples, the fibre diameter is 0.5mm and the fibre density is between about 90 fibres/cm$^2$ and about 220 fibres/cm$^2$, more preferably between about 90 fibres/cm$^2$ and about 170 fibres/cm$^2$, more preferably between about 90 fibres/cm² and about 140 fibres/cm².

In examples, the fibre diameter is 0.6mm and the fibre density is between about 70 fibres/cm² and about 170 fibres/cm², more preferably between about 80 fibres/cm² and about 140 fibres/cm².

In examples, the fibre diameter is 0.7mm and the fibre density is between about 60 fibres/cm² and about 130 fibres/cm², more preferably between about 70 fibres/cm² and about 130 fibres/cm².

In examples, the fibre diameter is 0.8mm and the fibre density is between about 50 fibres/cm² and about 110 fibres/cm², more preferably between about 60 fibres/cm² and about 110 fibres/cm², more preferably between about 70 fibres/cm² and about 110 fibres/cm².

In examples, the fibre diameter is 0.9mm and the fibre density is between about 50 fibres/cm² and about 90 fibres/cm², more preferably between about 60 fibres/cm² and about 90 fibres/cm², more preferably about 80 fibres/cm².

In specific examples, the envelope may have a cross-sectional area at the end support of between about 7 cm² and about 180 cm². In such examples, the plurality of fibres may comprise between about 500 fibres and about 60,000 fibres.

*Growing Area - Hollow Fibres*

According to another aspect the present invention may be characterised in that the fibre distribution may be such that the growing area (area within the envelope not occupied by fibres) is between about 43% and about 91%.

In examples, the fibre diameter is 0.3mm and the growing area is between about 77% and about 91%, more preferably between about 84% and about 91%, more preferably between about 87% and about 91%.

In examples, the fibre diameter is 0.4mm and the growing area is between about 62% and about 86%, more preferably between about 62% and about 77%, more preferably between about 81% and about 86%.

In examples, the fibre diameter is 0.5mm and the growing area is between about 57% and about 82%, more preferably between about 67% and about 82%, more preferably between about 73% and about 82%.

In examples, the fibre diameter is 0.6mm and the growing area is between about 52% and about 80%, more preferably between about 52% and about 80%, more preferably between about 60% and about 77%.

In examples, the fibre diameter is 0.7mm and the growing area is between about 50% and about 77%, more preferably between about 50% and about 73%.

In examples, the fibre diameter is 0.8mm and the growing area is between about 45% and about 75%, more preferably between about 45% and about 70%, more preferably between about 45% and about 65%.

In examples, the fibre diameter is 0.9mm and the growing area is between about 43% and about 68%, more preferably between about 43% and about 62%, more preferably about 49%.

In examples, the fibre diameter is 1mm and the growing area is between about 45% and about 61%.

Note that the above ranges correspond to the preferred ranges of the FDP for each diameter of fibre.

*Fibre Distribution Parameter (FDP) -Solid Fibres*

In some examples, the plurality of fibres are solid fibres. In such examples, during cell culturing a cell culture media may be introduced into, or circulated through, the cavity around the plurality of solid fibres and the cells growing on the outer surfaces of the solid fibres. In such examples the MDD is still applicable because nutrients in the cell culture media diffuse in the opposite direction - from the outside of the cell mass towards the surface of the solid fibre to feed cells on the fibre surface. However, the fibre spacing is preferably larger than with hollow fibres of the same diameter because there need to be channels extending along the housing through which the cell culture media can circulate as the cells grow into the spaces between the solid fibres. The size of these channels is preferably about 100 microns, which is large enough to permit cell culture media to circulate, and not so large as to reduce the capacity of the meat culture construct.

Accordingly, in examples the solid fibres are spaced by two times the MDD plus an additional spacing of 0.1mm to ensure that fluid channels remain during cell culturing. Table 3 shows the maximum fibre density that can be achieved when fibres are packed as efficiently as possible and separated by two times the MDD (where the MDD is 100 microns, 200 microns, 250 microns, and 300 microns) plus 0.1mm (100 microns).

*Table 3 - Maximum Number of Fibres per square centimetre by solid fibre diameter and MDD + 0.1mm fluid channel spacing*

| MDD (mm) | Solid Fibre Diameter (mm) | | | | | | | | | |
|---|---|---|---|---|---|---|---|---|---|---|
| | 0.2 | 0.3 | 0.4 | 0.5 | 0.6 | 0.7 | 0.8 | 0.9 | 1.0 | 1.5 |
| 0.1 | 429 | 304 | 216 | 168 | 126 | 105 | 85 | 68 | 56 | 30 |
| 0.2 | 216 | 168 | 126 | 105 | 85 | 68 | 56 | 52 | 42 | 23 |

(continued)

| MDD (mm) | Solid Fibre Diameter (mm) | | | | | | | | | |
|---|---|---|---|---|---|---|---|---|---|---|
| | **0.2** | **0.3** | **0.4** | **0.5** | **0.6** | **0.7** | **0.8** | **0.9** | **1.0** | **1.5** |
| 0.25 | 168 | 126 | 105 | 85 | 68 | 56 | 52 | 42 | 39 | 20 |
| 0.3 | 121 | 105 | 85 | 68 | 56 | 52 | 42 | 39 | 30 | 20 |

In addition, the parameters of cell adhesion and robustness as described above with reference to hollow fibres also apply to solid fibres. Accordingly, a minimum fibre diameter of 0.3mm is applied.

The inventors have realised that the preferred range of the FDP for solid fibres should also be informed by the MDD, channel spacing, and the minimum diameter of the fibres for cell adhesion and robustness.

The minimum fibre diameter of 0.3mm, and the maximum fibre densities of the fibres as shown in Table 2, broadly corresponds to the preferred range of the FDP - between 9 cm$^3$ and 24 cm$^3$. Accordingly, the meat culture construct with a FDP of between 9 cm$^3$ and 24 cm$^3$ provides advantages in terms of the balance between seeding length and growing area, and also accounts for the MDD, fibre surface curvature for cell adhesion, and the robustness of the fibres.

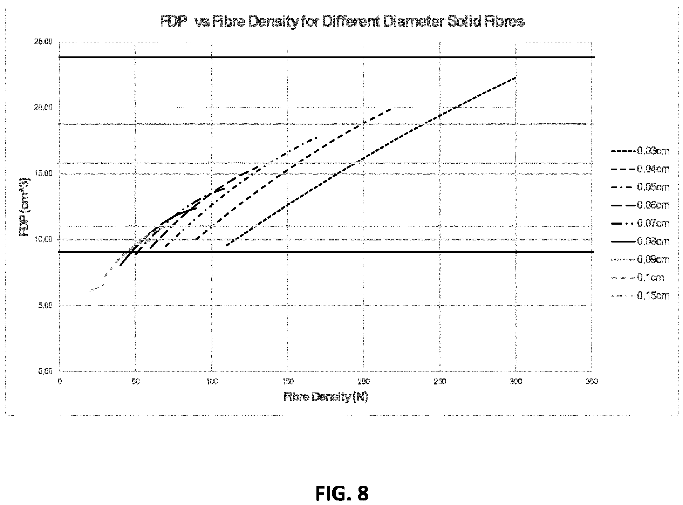

This is illustrated in FIG. 8 and Table 4. Fig. 8 shows the FDP for solid fibres having a diameter greater than 0.3mm with the curves truncated within the fibre densities shown in Table 3 above (minimum fibre density = 0.1mm MDD, maximum fibre density = 0.3mm MDD). The preferred ranges of FDP are indicated by the horizontal lines.

Table 4 provides the data on which the graph of FIG. 8 is based, showing the fibre densities for each diameter of fibre with the FDP. This provides preferred values and ranges of fibre density for each diameter of fibre. In Table 4, the lighter grey highlighted FDP values indicate the preferable range of 9 cm$^3$ to 24 cm$^3$. The darker grey highlighted FDP values indicate more preferred ranges. It can be seen that the FDP and fibre densities for 1.5mm fibres falls outside of the preferred range - these fibres are too large to provide the balance between seeding length and growing area. Accordingly, it can be seen that the preferred range of fibre diameters for the meat culture construct is 0.3 mm to 1 mm, more preferably 0.3 mm to 0.9 mm, more preferably 0.3 mm to 0.8 mm, more preferably 0.4 mm to 0.8mm, more preferably 0.5 mm to 0.8 mm, more preferably 0.5 mm to 0.7 mm.

| Fibre Density (N) | Fibre Diameters | | | | | | | | |
|---|---|---|---|---|---|---|---|---|---|
| | 0.3mm | 0.4mm | 0.5mm | 0.6mm | 0.7mm | 0.8mm | 0.9mm | 1mm | 1.5mm |
| 1 | | | | | | | | | |
| 10 | | | | | | | | | |
| 20 | | | | | | | | | 6.09 |
| 30 | | | | | | | | 7.20 | 6.64 |
| 40 | | | | | | 8.03 | 8.43 | 8.62 | |
| 50 | | | | | 8.88 | 9.41 | 9.64 | 9.54 | |
| 60 | | | | 9.39 | 10.15 | 10.53 | 10.49 | 9.97 | |
| 70 | | | 9.48 | 10.58 | 11.25 | 11.40 | 10.98 | | |
| 80 | | | 10.59 | 11.67 | 12.18 | 12.02 | | | |
| 90 | | 10.03 | 11.64 | 12.65 | 12.94 | 12.39 | | | |
| 100 | | 10.99 | 12.62 | 13.52 | 13.53 | | | | |
| 110 | 9.56 | 11.91 | 13.55 | 14.29 | 13.95 | | | | |
| 120 | 10.35 | 12.81 | 14.41 | 14.94 | | | | | |
| 130 | 11.13 | 13.67 | 15.21 | 15.50 | | | | | |
| 140 | 11.89 | 14.50 | 15.95 | | | | | | |
| 150 | 12.64 | 15.30 | 16.62 | | | | | | |
| 160 | 13.37 | 16.06 | 17.24 | | | | | | |
| 170 | 14.10 | 16.80 | 17.79 | | | | | | |
| 180 | 14.81 | 17.50 | | | | | | | |
| 190 | 15.50 | 18.18 | | | | | | | |
| 200 | 16.18 | 18.82 | | | | | | | |
| 210 | 16.85 | 19.43 | | | | | | | |
| 220 | 17.51 | 20.00 | | | | | | | |
| 230 | 18.15 | | | | | | | | |
| 240 | 18.78 | | | | | | | | |
| 250 | 19.40 | | | | | | | | |
| 260 | 20.00 | | | | | | | | |
| 270 | 20.59 | | | | | | | | |
| 280 | 21.17 | | | | | | | | |
| 290 | 21.73 | | | | | | | | |
| 300 | 22.28 | | | | | | | | |
| 310 | | | | | | | | | |
| 320 | | | | | | | | | |

*Table 4 - Table 2 – FDP by solid fibre diameter and fibre density (N = fibre density)*

As shown in FIG. 8 and Table 4, in examples, the FDP is preferably between 10 cm$^3$ and 18 cm$^3$. In particular, the fibre diameter may be between 0.5mm and 0.8mm, with the FDP being between 10 cm$^3$ and 18 cm$^3$. This narrower range of FDP provides advantages in terms of the balance between seeding length and growing area, as well as robust fibres that can be packed at an efficient density based on MDD. Accordingly, a meat culture construct having a fibre distribution with a FDP of between 10 cm$^3$ and 18 cm$^3$ provides improved meat culturing in contrast to known fibre bioreactors that are generally optimised by reducing fibre diameter and increasing the number of fibres (fibre density).

In examples, the FDP is preferably between 11 cm$^3$ and 16 cm$^3$. In particular, the solid fibre diameter may be between 0.5mm and 0.7mm, the FDP being between 11 cm$^3$ and 16 cm$^3$. This narrower range of FDP provides advantages in terms of the balance between seeding length and growing area, as well as robust fibres that can be packed at an efficient density based on MDD. Accordingly, a meat culture construct having a fibre distribution with a FDP of between 11 cm$^3$ and 16 cm$^3$ provides improved meat culturing in contrast to known fibre bioreactors that are generally optimised by reducing fibre diameter and increasing the number of fibres (fibre density).

In examples, the fibre diameter of the plurality of fibres is at least 0.3mm. In examples, the outer fibre diameter of the plurality of fibres is between 0.3mm and 1 mm, for example between 0.4mm and 0.9mm, for example between 0.5mm and 0.8mm, for example one of 0.3mm, 0.4mm, 0.5mm, 0.6mm, 0.7mm, 0.8mm, 0.9mm or 1mm.

In examples, some of the plurality of fibres may have different outer diameters to others. Fibres with different outer diameters may be distributed evenly through the envelope, or concentrated in one or more locations within the envelope, for example larger fibres may be concentrated towards a centre of the envelope.

In specific examples, the fibres have a diameter of 0.3mm and the meat culture construct has a FDP of between 9 $cm^3$ and 23 $cm^3$, more preferably between 10 $cm^3$ and 18 $cm^3$, more preferably between 11 $cm^3$ and 16 $cm^3$.

In specific examples, the fibres have a diameter of 0.4mm and the meat culture construct has a FDP of between 10 $cm^3$ and 20 $cm^3$, more preferably between 10 $cm^3$ and 18 $cm^3$, more preferably between 11 $cm^3$ and 16 $cm^3$.

In specific examples, the fibres have a diameter of 0.5mm and the meat culture construct has a FDP of between 9 $cm^3$ and 18 $cm^3$, more preferably between 10 $cm^3$ and 18 $cm^3$, more preferably between 11 $cm^3$ and 16 $cm^3$.

In specific examples, the fibres have a diameter of 0.6mm and the meat culture construct has a FDP of between 9 $cm^3$ and 16 $cm^3$, more preferably between 10 $cm^3$ and 16 $cm^3$ more preferably between 11 $cm^3$ and 16 $cm^3$.

In specific examples, the fibres have a diameter of 0.7mm and the meat culture construct has a FDP of between 10 $cm^3$ and 14 $cm^3$, more preferably between 11 $cm^3$ and 14 $cm^3$.

In specific examples, the fibres have a diameter of 0.8mm and the meat culture construct has a FDP of between 9 $cm^3$ and 13 $cm^3$, more preferably between 10 $cm^3$ and 13 $cm^3$, more preferably between 11 $cm^3$ and 13 $cm^3$.

In specific examples, the fibres have a diameter of 0.9mm and the meat culture construct has a FDP of between 9 $cm^3$ and 11 $cm^3$, more preferably between 10 $cm^3$ and 11 $cm^3$, more preferably about 11 $cm^3$.

In specific examples, the fibres have a diameter of 1mm and the meat culture construct has a FDP of between 9 $cm^3$ and 10 $cm^3$, more preferably about 9 $cm^3$.

*Fibre Density - Solid Fibres*

It will be appreciated that the advantageous range FDP can be used to define an advantageous range of fibre density - the number of fibres per unit area. However, the FDP advantageously defines the characteristics of the fibre distribution irrespective of fibre diameter, and so has advantages over defining the fibre density itself, which varies more based on fibre diameter.

Nevertheless, according to another aspect the present invention may be characterised in that the fibre density is preferably between about 50 fibres/$cm^2$ and about 300 fibres/$cm^2$. In examples, the fibre density is between about 60 fibres/$cm^2$ and about 220 fibres/$cm^2$, for example between about 70 fibres/$cm^2$ and about 190 fibres/$cm^2$. This is shown in Table 4.

In an example, the fibre diameter is between 0.3mm and 1mm and the fibre density is preferably between about 50 fibres/$cm^2$ and about 300 fibres/$cm^2$. In examples, the fibre density is between about 60 fibres/$cm^2$ and about 220 fibres/$cm^2$, for example between about 70 fibres/$cm^2$ and about 190 fibres/$cm^2$.

In another example the fibre diameter is between 0.5mm and 0.8mm and the fibre density is between about 50 fibres/$cm^2$ and about 170 fibres/$cm^2$, more preferably between about 60 fibres/$cm^2$ and about 170 fibres/$cm^2$, more preferably between about 70 fiibres/$cm^2$ and about 140 fibres/$cm^2$.

In another example, the fibre diameter is between 0.5mm and 0.7mm and the fibre density is between about 60 fibres/$cm^2$ and about 170 fibres/$cm^2$, more preferably between about 60 fibres/$cm^2$ and about 180 fibres/$cm^2$, more preferably between about 70 fiibres/$cm^2$ and about 140 fibres/$cm^2$.

In examples, the fibre diameter is 0.3mm and the fibre density is between about 110 fibres/$cm^2$ and about 300 fibres/$cm^2$, more preferably between about 120 fibres/$cm^2$ and about 220 fibres/$cm^2$, more preferably between about 130 fibres/$cm^2$ and about 190 fibres/$cm^2$.

In examples, the fibre diameter is 0.4mm and the fibre density is between about 90 fibres/$cm^2$ and about 220 fibres/$cm^2$, more preferably between about 90 fibres/$cm^2$ and about 180 fibres/$cm^2$, more preferably between about 110 fibres/$cm^2$ and about 150 fibres/$cm^2$.

In examples, the fibre diameter is 0.5mm and the fibre density is between about 70 fibres/$cm^2$ and about 170 fibres/$cm^2$, more preferably between about 80 fibres/$cm^2$ and about 170 fibres/$cm^2$, more preferably between about 90 fibres/$cm^2$ and about 140 fibres/$cm^2$.

In examples, the fibre diameter is 0.6mm and the fibre density is between about 60 fibres/$cm^2$ and about 130 fibres/$cm^2$, more preferably between about 70 fibres/$cm^2$ and about 130 fibres/$cm^2$, more preferably between about 80 fibres/$cm^2$ and about 130 fibres/$cm^2$.

In examples, the fibre diameter is 0.7mm and the fibre density is between about 60 fibres/$cm^2$ and about 110 fibres/$cm^2$, more preferably between about 70 fibres/$cm^2$ and about 130 fibres/$cm^2$.

In examples, the fibre diameter is 0.8mm and the fibre density is between about 50 fibres/$cm^2$ and about 90 fibres/$cm^2$, more preferably between about 60 fibres/$cm^2$ and about 90 fibres/$cm^2$, more preferably between about 70 fibres/$cm^2$ and about 90 fibres/$cm^2$.

In examples, the fibre diameter is 0.9mm and the fibre density is between about 50 fibres/$cm^2$ and about 70 fibres/$cm^2$, more preferably between about 60 fibres/$cm^2$ and about 70 fibres/$cm^2$.

In examples, the fibre diameter is 1mm and the fibre density is between about 50 fiibres/$cm^2$ and about 60 fibres/$cm^2$.

In specific examples, the envelope may have a cross-sectional area at the end support of between about 7 $cm^2$

and about 180 cm$^2$. In such examples, the plurality of fibres may comprise between about 350 fibres and about 54,000 fibres.

*Growing Area - Solid Fibres*

According to another aspect the present invention may be characterised in that the fibre distribution may be such that the growing area (area within the envelope not occupied by fibres) is between about 55% and about 92%.

In examples, the fibre diameter is 0.3mm and the growing area is between about 79% and about 92%, more preferably between about 84% and about 92%, more preferably between about 87% and about 92%.

In examples, the fibre diameter is 0.4mm and the growing area is between about 72% and about 89%, more preferably between about 77% and about 89%, more preferably between about 81% and about 86%.

In examples, the fibre diameter is 0.5mm and the growing area is between about 67% and about 86%, more preferably between about 67% and about 84%, more preferably between about 73% and about 82%.

In examples, the fibre diameter is 0.6mm and the growing area is between about 63% and about 83%, more preferably between about 63% and about 80%, more preferably between about 63% and about 77%.

In examples, the fibre diameter is 0.7mm and the growing area is between about 58% and about 81%, more preferably between about 58% and about 77%, more preferably between about 58% and about 73%.

In examples, the fibre diameter is 0.8mm and the growing area is between about 55% and about 75%, more preferably between about 55% and about 70%, more preferably between about 55% and about 65%.

In examples, the fibre diameter is 0.9mm and the growing area is between about 55% and about 68%, more preferably between about 55% and about 62%.

In examples, the fibre diameter is 1mm and the growing area is between about 53% and about 61%.

Note that the above ranges correspond to the preferred ranges of the FDP for each diameter of fibre.

*Construction of the Meat Culture Construct*

The fibre density and growing area as described above are defined within a two dimensional plane within the envelope. In some examples there may be more than one envelope defined within a meat culture bioreactor module, either in the same end support of a different end support. In addition, the meat culture construct may comprise more than one envelope. For example, a first set of the plurality of fibres may be provided in a first envelope and a second set of the plurality of fibres may be provided in a second envelope that is adjacent to, or fully or partially surrounding, the first envelope (in the same plane, e.g., at the end support). The first and/or second envelope (and/or any further envelope) may have a fibre distribution as set out above. The fibre density may be referred to as the envelope density - the density of the fibres within the envelope.

It will be appreciated that the above description relates to a two-dimensional cross-section of the meat culture construct, specifically at a plane coincident with the end support, and the fibres extend from the end support through the housing of the meat culture bioreactor module. In some examples the fibres maintain their fibre distribution through the housing, for example due to the stiffness of the fibres, and/or due to further supports along the housing, and/or due to the opposite ends of the fibres being supported in the same fibre distribution. In some examples the fibres are held under tension to maintain the fibre distribution. However, in other examples the fibre distribution may be non-uniform through the housing.

In examples, the envelope may extend through the housing and define the volume into which the plurality of fibres extend from the end support. However, the cross-sectional size and/or shape of the envelope may vary along the housing.

In examples, a cross-sectional area of the envelope is smaller than the cross-sectional area of the end support. That is, the fibres are arranged within only a part of the end support. For example, the plurality of fibres may be concentrated or bunched towards a centre of the end support, or offset from the centre of the end support. In some examples, there may be more than one envelope defined, for example by two distinct bundles of fibres embedded in the same end support.

In other examples, the envelope may have a cross-sectional area that substantially matches the end support, for example the fibre distribution may be arranged across substantially an entire surface of the end support that faces into the housing when the meat culture bioreactor is assembled.

In examples, at the end support the fibre density is substantially uniform (even) across the envelope. That is, in these examples the plurality of fibres are substantially evenly spaced across the envelope.

In other examples, the fibre density varies across the envelope. That is, the fibres in a first area of the envelope may have a first fibre density, and fibres in a second area of the envelope may have a second fibre density. The fibre density may vary linearly or non-linearly across the envelope. In one example, the fibre density at a centre of the envelope may be higher than a fibre density at or towards an edge of the envelope. Such an arrangement may improve the seeding of cells on the fibres by providing increased space between outer fibres that allows the cell suspension to reach the fibres in the middle of the envelope. In such examples the FDP, fibre densities and growing areas described above may be based on an average value for the entire fibre distribution within the envelope.

In one example, a first set of the plurality of fibres may be arranged within, and surrounded by, a second set of the plurality of fibres, for example in a concentric arrangement. In such an example, the fibre density of the first set of the plurality of fibres may be higher than the fibre density of the second set of the plurality of fibres. In this example the fibres arranged in the centre of the fibre distribution may have a higher fibre density than fibres arranged towards the edge(s) of the fibre distribution.

In examples, the plurality of fibres may be aligned. In particular, the plurality of fibres may be parallel where they are embedded in the end support. In examples, the plurality of fibres are straight fibres. The straight fibres may extend parallel to the housing. In other examples, the plurality of fibres may be at least partly wavy, spiral, or zig-zag along their length (specifically the length that extends into the housing).

In examples, the meat culture construct may further comprise a second end support and opposite ends of the plurality of fibres are embedded in the second end support. The end support and the second end support may be receivable at opposite ends of the housing such that the plurality of fibres extend between the end support and the second end support within the housing.

In examples, the or each end support comprises a sealing material, which may be a resin or glue or potting material. The sealing material can be introduced between the plurality of fibres in a fluid form, for example by a centrifuge, and then cured to form the end support. After curing an end of the end support may be cut off to expose ends of the fibres.

In other examples, the or each end support may clamp or otherwise hold the plurality of fibres, without a sealing material. For example, the plurality of fibres may be pushed through openings formed in the end support, or pushed through a soft ductile material, or clamped or crimped within a sleeve. In some examples, the plurality of fibres may be sealed at the end support, and clamped at the second end support.

In alternative examples the opposite ends of the plurality of fibres (opposite to the end support) may be free. That is, the plurality of fibres may only be attached at the end support, and may extend freely into the cavity. Such an example may be advantageous where the meat culture bioreactor module is held vertically during cell culturing.

*Fibres*

As described above, the fibres may be hollow or solid fibres. In examples, the hollow fibres may be porous.

In certain embodiments, the one or more fibres comprise a polymer. In certain embodiments, the one or more fibres are biodegradable and/or edible.

Polymers that may be used to form the fibres may be any polymer suitable for culturing and/or maintenance of cells. Suitable polymers include biodegradable polymers. The polymer may be a biocompatible polymer. Biodegradable polymers are any polymers that may be broken down by biological systems, such as polymers that can be broken down into harmless products by the action of living organisms. Biocompatible polymers are, along with any metabolites or degradation products thereof, generally nontoxic to cells or to a recipient (such as a human or animal) and do not cause any significant adverse effects to cells or a recipient at concentrations resulting from the degradation of the polymers. Generally speaking, biocompatible polymers are polymers that do not elicit result in negative effects on cell health or in a recipient. As one use for the fibres described herein is the production of comestible products, biocompatible and/or biodegradable polymers may be advantageous if the fibres or part thereof is consumed (for example, ingested) by a person.

Biodegradable polymers include linear aliphatic polyesters such as polylactic acid, polyglycolic acid, polycaprolactone, polyhydroxybutyrate, polyhydroxyvalerate and their copolymers within the aliphatic polyester family such as poly(lactic-co-glycolic acid) and poly(glycolic acid-co-caprolactone); copolymers of linear aliphatic polyesters and other polymers such as poly(glycolic acid-co-trimethylene carbonate) copolymers, poly(lactic acid-co-lysine) copolymers, tyrosine-based polyarylates or polyiminocarbonates or polycarbonates, poly(lactide-urethane) and poly(ester-amide) polymers; polyanhydrides such as poly(sebacic anhydride); polyorthoesters such as 3,9-diethyidiene-2,4,8,10-tetraoxaspiro-5,5-undecane based polymers; poly(ester-ether) such as poly-p-dioxanone; polyamides, poly(amide-enamines) and poly(amido amine) dendrimers; and phosphorus-based polymers such as polyphosphazene and poly[bis(carboxylactophenoxy)] phosphazene.

The polymers may be edible polymers. Edible polymers refers to any polymer that is acceptable for use in an edible product.

Examples of edible polymers include polyvinyl alcohol, carboxyvinyl polymer, hydroxypropylmethylcellulose, hydroxyethylcellulose, methylcellulose, ethylcellulose, low-substituted hydroxypropylcellulose, crystalline cellulose, carboxymethylcellulose sodium, a synthetic polymer compound such as carboxymethylcellulose calcium, carboxymethylcellulose and carboxymethylstarch sodium, sodium alginate, dextran, casein, pullulan, pectin, guar gum, xanthan gum, tragacanth gum, acacia gum, zein, gelatin, chitin and chitosan, silk, fibrin and polymer compounds obtained from natural products such as starch or soybean. Edible polymers may include edible proteins from animal or plant sources. For example such edible proteins may be derived from legumes such as chickpea or pea. Edible polymers may also be derivable from waste food such as potatoes.

The use of an edible polymer may provide fibres and products, including the fibres which are edible. For example, a cell culture grown on the fibres may provide for an edible product that does not require the removal of the fibres before consumption.

The fibres may be a digestible polymer. "Digestible" refers to a material that, when eaten by a subject, can be broken down into compounds that can be absorbed and used as nutrients or eliminated by the subject's body. Digestible polymers include BCS, polylactic acids (PLAs), synthetic polyamides, polycarbonates, polyisocyanurates (PIRs), polyurethanes, polyethers, proteins, polysaccharides (such as starches), polylactones, polylactams or glycols.

In some examples, the fibres include poly(lactic-co-glycolic acid) (PLGA). For example, the fibres may include 10% PLGA.

In some examples, the fibres include a reusable polymer. A reusable polymer refers to a polymer that does not degrade over time or due to use in culturing cells. Reusable polymers may provide a meat culture construct that can be washed and used multiple times. In some examples, the fibres are hydrophobic. In some examples, the fibres are washable. Washable refers to hollow fibres that can be washed and/or sterilised without sustaining damage or loss of function. Fibres with such properties may reduce waste and costs.

In some examples, the polymer may comprise cellulose or cellulosic polymers or mixtures of both, polysulfone, polypropylene, acrylonitrile butadiene styrene (ABS), polycarbonate, polyethylene, or mixtures thereof. Suitable polymers include biodegradable polymers. Biodegradable polymers are any polymers that may be broken down by biological systems, such as polymers that can be broken down into harmless products by the action of living organisms.

In some examples, the polymer comprises polystyrene. The polymer can comprise polystyrene and a second polymer. The second polymer can be selected from the group consisting of polycarbonate, polypropylene, polyvinylidene fluoride, polycaprolactone, polysulfone, cellulose acetate, cellulose triacetate, polyethersulfone, polyphenylenesulfon, polymethyl methacrylate, acrylonitrile butadiene styrene or mixtures thereof. The composition can comprise polystyrene and a second polymer in a wt. % ratio of about 5:95, 10:90, 15:85, 20:80, 25:75, 30:70, 35:65, 40:60, 45:55, 50:50, 55:45, 60:40, 65:35, 70:30, 75:25, 80:20, 85:15, 90:10 or 95:5.

In some examples, the polymer comprises polysulfone. The polymer can comprise polysulfone and a second polymer. The second polymer can be selected from the group consisting of polystyrene, polycarbonate, polypropylene, polyvinylidene fluoride, polycaprolactone, cellulose acetate, cellulose triacetate, polyethersulfone, polyphenylenesulfon, polymethyl methacrylate, acrylonitrile butadiene styrene or mixtures thereof. The composition can comprise polysulfone and a second polymer in a wt. % ratio of about 5:95 to about 95:5 (e.g. about 10:90 to about 90:10), more preferably about 20:80 to about 80:20, and even more preferably about 25:75 to about 75:25. Polysulfone and the second polymer may be present in a wt. % ratio of 30:70, 35:65, 40:60, 45:55, 50:50, 55:45, 60:40, 65:35, 70:30.

Preferably, the polymer comprises polystyrene and/or polysulfone. Preferably, the polymer comprises polystyrene and polysulfone in a wt. % ratio of about 5:95 to about 95:5 (e.g. about 10:90 to about 90:10), more preferably about 20:80 to about 80:20, and even more preferably about 25:75 to about 75:25. Polystyrene and polysulfone may be present in a wt. % ratio of 30:70, 35:65, 40:60, 45:55, 50:50, 55:45, 60:40, 65:35, 70:30. Preferably, the composition comprises about 80 wt. % of polystyrene and about 20 wt. % of polysulfone, or about 60 wt. % of polystyrene and about 40 wt. %.

In some examples, the polymer comprises high impact polystyrene (HIPS). The polymer can comprise HIPS and a second polymer. The second polymer can be selected from the group consisting of polystyrene, polycarbonate, polypropylene, polyvinylidene fluoride, polycaprolactone, polysulfone, cellulose acetate, cellulose triacetate, polyethersulfone, polyphenylenesulfon, polymethyl methacrylate, acrylonitrile butadiene styrene or mixtures thereof. The composition can comprise HIPS and a second polymer in a wt. % ratio of about 5:95 to about 95:5 (e.g. about 10:90 to about 90:10), more preferably about 20:80 to about 80:20, and even more preferably about 25:75 to about 75:25. HIPS and the second polymer may be present in a wt. % ratio of 30:70, 35:65, 40:60, 45:55, 50:50, 55:45, 60:40, 65:35, 70:30.

The polymer may be present in the composition in an amount of from about 5 wt. % to about 50 wt. %, preferably from about 10 wt. % to about 40 wt. %, from about 15 wt. % to about 30 wt. %.

The wt. % ratio of the non-toxic solvent and the polymer present in the composition may vary depending on the components present and solubility of the polymer thereof. In some examples, the polymer and non-toxic solvent may be present in the composition in a wt. % ratio of about 1: 1 to 10, preferably about 1: 2 to 7, more preferably about 1:3 to 6.

Hollow fibres may be themselves be solid or semi-solid substrates having openings or apertures (pores), which allow components of cell culture media, such as metabolites, nutrients and gases (e.g. oxygen), to be delivered to cells attached to the outer surfaces of the hollow fibres.

Fibres described herein allow the growth of cells on the outer surface of the fibres. Thus the fibres described herein act as a 3-dimensional matrix that allows for the culture and maintenance of cells in a 3-dimensional architecture.

The fibres may have a Young's modulus of at least 1000 Pa. In some examples, the fibres have a Young's modulus from 1000 Pa to 1000000000 Pa. In some examples, the fibres have a Young's modulus between 8000 Pa to about 20000 Pa.

For example, the fibres may have a Young's modulus of around 115 MPa.

For hollow fibres, the lumens of each hollow fibre act as conduits to transport cell culture medium to the cells located on the outer surface of the hollow fibres, as well as transporting waste products from the cells. The density of pores on each hollow fibre may be at least 1 pore/mm2. For example, at least 1, 2, 3, 4, 5, 6, 7, 8, 9, 10, 20, 30, 40, 50, 60, 70, 80, 90, 100, 110, 120, 130, 140, 150, 160, 170, 180, 190, 200, 210, 220, 230, 240, 250, 260, 270, 280, 290, 300 pores/mm2.

In some examples, the pore density of the hollow fibres may be around 210 pores/mm$^2$.

Hollow fibres may be produced by any method, many of which are known in the field. For example, melting spinning, solution spinning, wet spinning, gel spinning, dry-wet spinning, liquid crystal spinning, dispersion spinning, reaction spinning and electrospinning.

In some examples, the hollow fibres may be fibres as described in Luetchford, Kim A., et al. "Next generation in vitro liver model design: Combining a permeable polystyrene membrane with a transdifferentiated cell line." Journal of membrane science 565 (2018): 425-438, which is incorporated herein in its entirety.

*Meat Culture Bioreactor Module*

According to the present invention there is also provided a meat culture bioreactor module comprising the meat culture construct described above and a housing having a cavity within which the meat culture construct is receivable such that the plurality of fibres extend into the cavity.

In examples, the housing and the cavity may be elongate and the plurality of fibres may extend generally longitudinally along the cavity when the meat culture construct is received in the cavity.

In examples, the cavity comprises a cross-sectional area through which the plurality of fibres extend. The cross-sectional area may be circular, square, rectangular, triangular, or other shape. The cross-sectional area and/or shape may be constant along the length of the housing, or varying along the length of the housing. The cross-sectional area and/or shape may be constant along a length of the housing that corresponds to the length of the plurality of fibres such that the cross-sectional area/shape of the cavity is constant in the portion where the plurality of fibres are received.

In examples, the housing may have an inlet for introduction of a cell suspension into the cavity. The inlet may extend perpendicularly to the longitudinal direction of the housing, for example from a side surface of the housing. In examples, the housing may additionally include an outlet for removal of fluids from the cavity, for example for removal of the cell suspension. During seeding the cell suspension may be circulated through the cavity via the inlet and outlet. During cell growth (culturing) a cell culture medium may be circulated through the cavity via the inlet and the outlet.

In examples, the inlet is arranged at or towards a first end of the housing. The outlet may be arranged at or towards a second end of the housing. Advantageously, as described below, the housing may be inclined during cell seeding. The housing may be inclined such that the inlet is arranged higher than the outlet. This arrangement ensures that the introduced cell suspension flows over substantially all of the plurality of fibres during cell seeding.

In examples, the housing may be inclined from a horizontal position, for example at an angle of between 5 degrees and 85 degrees from the horizontal, for example between 5 degrees and 45 degrees from the horizontal, for example between 8 degrees and 40 degrees from the horizontal, for example between 10 degrees and 35 degrees from the horizontal, for example between 15 degrees and 25 degrees from the horizontal, for example about 20 degrees from the horizontal.

In examples, as described above, the end support and optional second end support may comprise a sealing material. In examples, the end support and the optional second end support may comprise a sleeve within which the sealing material is provided. The sleeve may be attachable (sealable) to the housing. The sleeve may include a seal, for example a gasket, face seal, or O-ring on an outer surface of the sleeve (end surface or side surface). The seal may act to seal the seal within an opening of the housing. This provides for simple removal of the meat culture construct from the housing, which may be advantageous for harvesting cultured meat from the bioreactor module.

In examples, the housing may be openable. Specifically, the housing may comprise an openable portion for accessing the cavity. The meat culture construct may be removable from the housing by opening the openable portion. Additionally, the meat culture construct may be insertable into the housing by opening the openable portion. Therefore, the openable portion may provide a simple method of assembling the meat culture bioreactor module, and/or of removing the meat culture construct from the housing for harvesting cultured meat.

In examples, the openable portion may extend along at least 50% of the cavity in the longitudinal direction, for example at least 75%, for example at least 90%. In examples, the openable portion extends along the housing by at least the length of the plurality of fibres within the cavity, for example the entire length of the housing.

In examples, the housing comprises a first housing part and a second housing part. The cavity may comprise a first portion formed in the first housing part and a second portion formed in the second housing part. Therefore, the cavity is defined when the first and second housing parts are in a closed position. In examples, the first and second portions of the cavity may each be semi-cylindrical such that the cavity is cylindrical.

In examples, the openable portion is hingedly connected to the remainder of the housing. In examples, the hinged connection extends parallel to the longitudinal direction of the housing and cavity. Accordingly, the openable portion provides side-access into the cavity for removal of the meat culture construct in a direction perpendicular to the longitudinal direction of the housing (and the plurality of fibres).

In examples, as mentioned above, the meat culture construct comprises an end support, which comprise a sleeve. In examples, the end support, in particular the sleeve, may be clamped between the openable portion and the remainder of the housing so as to form a seal between the housing and the end support. In this example, when the openable portion is opened the entire meat culture construct can be removed by separating the end support from the housing. Advantageously, the cultured meat can be removed from the housing by lifting the end support, avoiding the need to contact the cultured meat and/or the plurality of fibres.

In some examples, the end support may comprise more than one bundle of fibres embedded in, and extending from, the end support. In various examples the different bundles of fibres may be arranged in the same or different housings when the meat culture construct is received in the housing.

*Meat Culturing System*

According to the present invention there is also provided a meat culturing system comprising the meat culture bioreactor module and a base unit, the meat culture bioreactor module being detachable from the base unit.

In examples, the base unit is adapted to hold the meat culture bioreactor module such that the housing is inclined from a horizontal position.

In examples, the base unit comprises an actuator arranged to rotate the housing. In examples, the meat culture bioreactor module may be rotatable within the base unit. In particular, the meat culture bioreactor module may be rotatable about a rotation axis parallel to a longitudinal axis of the housing. The longitudinal axis of the housing may be central within the housing, and the rotation axis may be offset from the longitudinal axis of the housing.

In examples, the base unit and actuator are arranged to rotate the housing while holding the housing at the inclined angle. Advantageously, circulating the cell suspension around the plurality of fibres of the construct when at an angle brings cells into contact with the external surface of the fibres and aids with cell adhesion. The rotational motion of the construct mixes the fluid around the external surfaces of the fibres and ensures a uniform distribution of cells. The rotational speed of the housing during seeding is selected to provide this mixing, and is limited to prevent forces that could impede adhesion of the cells to the fibres.

In examples, the meat culturing system further comprises a cell supply system in fluid communication with an inlet of the meat culture bioreactor module for introducing a cell suspension into the cavity. The cell supply system may be connected to an inlet of the housing. The housing may also include an outlet, and the cell suspension may be circulated through the housing during cell seeding, and/or excess cell suspension may be removed from the housing after seeding.

In examples, the housing comprises an inlet port and an outlet port, and the meat culturing system comprises a pump system in fluid communication with the inlet port and the outlet port of the meat culture bioreactor module and operable to circulate a cell culture medium through the meat culture bioreactor module. The meat culturing system may be a perfusion system.

In examples, the plurality of fibres may comprise a plurality of hollow fibres. The plurality of hollow fibres may be sealed at the inlet port and at the outlet port. The pump system may be operable to circulate the cell culture medium through the plurality of hollow fibres. In examples, the inlet port and the outlet port each comprise a one-way valve.

The pump system may be any suitable pump. For example, the pump system may be a peristaltic pump system or vacuum pump system. The flow rate of cell culture media that is flowed through the meat culture bioreactor module may be controlled by the pump system. The flow rate may be selected depending on the number of fibres being used, the cells being cultured and/or the number of cells seeded onto the outer surface of the fibres.

For example, cell culture media may be pumped at a rate of at least 1 $\mu$L/hour/ fibre. That is to say, for each fibre used, the flow rate is 1 $\mu$L/hour. In some examples, the flow rate is at least 10 $\mu$L/hour/ fibre. For example, at least 10, 20, 30, 40, 50, 60, 70, 80, 90, 100, 200, 300, 400 $\mu$L/hour/ fibre. In some examples, the flow rate is from 100 to 300 $\mu$L/hour/ fibre. In some examples, the flow rate is between 240 to 280 $\mu$L/hour/ fibre. In some examples, the flow rate is about 267 $\mu$L/hour/ fibre.

The meat culture bioreactor system may further include monitoring apparatus. Monitoring apparatus refers to any apparatus suitable for determining and/or monitoring changes in one or more properties of cells being cultured and/or one or more properties of the cell culture media after having flowed through the meat culture bioreactor module (retentate).

For example, the monitoring apparatus may be a system for monitoring nutrient and/or metabolite content of the cell culture media entering the meat culture bioreactor module (the permeate for examples with hollow fibres) and/or retentate. As used herein, in examples with hollow fibres "permeate" refers to cell culture media that has passed through the pores of the hollow fibres (i.e. permeated through the hollow fibres). As used herein, retentate refers to cell culture media that flows through the meat culture bioreactor module. Nutrients that may be monitored may be any nutrient and/or metabolite that is included in the cell culture media used. For example, the nutrient and/or metabolite may be one or more of a saccharide (such as glucose), lactic acid (lactate), glutamine, glutamate, ammonia (ammonium ion), essential and non-essential amino acids, growth factors, albumin, attachment proteins, vitamins, oxygen, and or carbon dioxide or the like.

The concentrations of nutrients and/or metabolites may be compared to the initial concentration of the corresponding nutrients and/or metabolites in the cell culture media prior to culturing the cells or prior to being circulated through the meat culture bioreactor module. Changes in concentrations of nutrients and/or metabolites in the permeate and/or retentate in comparison to the initial cell culture media may provide information on the rate of proliferation, cell number and/or condition (e.g. viability and/or health) of the cells being cultured.

In some examples, systems for monitoring concentrations of nutrients and/or metabolites may be separate from the meat culture bioreactor module and system. For example, the methods may further include collecting a portion of the permeate and/or retentate at predetermined time points and determining the concentrations of nutrients and/or metabolites. In some examples, the meat culture bioreactor system may include a system for monitoring nutrients and/or metabolites that is connected directly to the meat culture bioreactor module.

Suitable methods for determining concentrations of nutrients and/or metabolites in cell culture media are well known in the field. For example, kits such as those provided by

Megazyme are available that include instructions therein. For example, for nutrients and/or metabolites such as saccharides (such as glucose), lactic acid (lactate), glutamine, glutamate, ammonia (ammonium ion). Alternatively or additionally HPLC and/or GC-MS may be used to determine concentrations of essential and non-essential amino acids, growth factors, albumin, attachment proteins. For example, monitors include those available from PreSens for oxygen and carbon dioxide.

In some examples, the monitoring apparatus may include a system for monitoring a concentration of dissolved oxygen in the permeate and/or retentate. The concentrations of dissolved oxygen may be compared to the initial concentration of dissolved oxygen in the cell culture media prior to culturing the cells or prior to being circulated through the meat culture bioreactor module. Changes in concentrations of dissolved oxygen in the permeate and/or retentate in comparison to the initial cell culture media may provide information on the rate of proliferation, cell number and/or condition (e.g. viability and/or health) of the cells being cultured.

Systems for determining the concentration of dissolved oxygen in cell culture media are well known and include, for example, PreSens flow-through cell and PreSens Fibox 4 oxygen reader.

In examples, the meat culturing system may further comprise a base unit adapted to support the meat culture bioreactor module. The support may comprise first and second fluid connectors that attach to the inlet port and the outlet port of the meat culture bioreactor module, respectively, when the meat culture bioreactor module is received in the base unit.

*Seeding Method*

According to the present invention there is also provided a method of seeding muscle cells and/or muscle cell precursors in the meat culture bioreactor module. The method comprises:

inclining the meat culture bioreactor module,

introducing a cell suspension into the housing, and

rotating the meat culture bioreactor module.

In examples, prior to seeding the muscle cells and/or muscle cell precursors are:

(i) washed with a composition comprising a cell detachment agent;

(ii) harvested in a culture media comprising a growth promotion agent;

(iii) centrifuged to form a pellet; and

(iv) resuspended in a culture media to form a cell suspension.

In examples, the housing may be inclined at an angle of between 5 degrees and 85 degrees from the horizontal, for example between 5 degrees and 45 degrees from the horizontal, for example between 8 degrees and 40 degrees from the horizontal, for example between 10 degrees and 35 degrees from the horizontal, for example between 15 degrees and 25 degrees from the horizontal, for example about 20 degrees from the horizontal.

Advantageously, inclining the meat culture bioreactor module (in particular the housing and meat culture construct) during cell seeding improves cell seeding on the external surfaces of the plurality of fibres. In particular, in examples the housing comprises an inlet at or towards one end of the housing which is in a raised position when the meat culture bioreactor module is inclined. When the cell suspension is introduced into the housing through the inlet the cell suspension will flow over the plurality fibres, providing improved seeding. At the same time, the fibre distribution within the housing, as described above, advantageously provides space (i.e., free volume) around the plurality of fibres that allows the cell suspension to reach substantially all of the plurality of fibres and obtain substantially even seeding of cells on the plurality of fibres.

The cells seeded onto the fibres include muscle cells and/or muscle cell precursors. Muscle cells include those cells making up contractile tissue of animals or cells that can differentiate into muscle cells. Muscle cells are derived from the mesodermal layer of embryonic germ cells. Mature muscle cells contain contractile filaments that move past each other and change the size of the cell. They are classified as skeletal, cardiac, or smooth muscles. As used herein, the term "cells that can differentiate into muscle cells" and "muscle cell precursors" refers to stem cells and muscle progenitor cells that can differentiate into muscle cells (e.g. mature muscle cells).

Muscle cells may include those cells normally found in muscle tissue, including smooth muscle cells, cardiac muscle cells, skeletal muscle cells (e.g., muscle fibres or myocytes, myoblasts, myotubes, etc.), and any combination thereof. Muscle cells may include myoblasts, myotubes, myofibrils, and/or satellite cells.

The cells may further include adipose or fat cells. Adipose or fat cells include any cell or group of cells composed in a fat tissue, including, for example, lipocytes, adipocytes, adipocyte precursors including, pre-adipocytes and mesenchymal stem cells.

The cells may be derived from any source animal. As the bioreactor systems described herein may be for use in making comestible products, the cells may not be derived from a human. In some examples, the cells may be derived from bovine, ovine, equine, porcine, caprine, avian, fish, insect, crustaceans, cephalopod, mollusc and/or camelid animals. Preferably the cells may be derived from a bovine, porcine, avian and/or ovine animal. For example, the cells may be derived from a cow, pig, chicken, fish, squid, insect, oyster and/or sheep.

The meat culture bioreactor module may be attachable to a base unit during cell seeding, and detached from the base unit after cell seeding. The base unit may hold the bioreactor meat culture module in the inclined position. Alternatively, the base unit may comprise a turning mechanism adapted to hold the meat culture bioreactor module in an inclined position during cell sending, and to turn the meat culture bioreactor module to a vertical or horizontal position for cell culturing.

In examples, the meat culture bioreactor module may be rotated within the base unit. In particular, the meat culture bioreactor module may be rotated about a rotation axis parallel to a longitudinal axis of the housing. The longitudinal axis of the housing may be central within the housing, and the rotation axis may be offset from the longitudinal axis of the housing. In examples, the base unit may comprise an actuator operable to rotate the meat culture bioreactor module. The actuator may be a tube rotator, such as a MACSmixTM available from Miltenyi Biotech.

The meat culture bioreactor module may be rotated during the initial attachment stage (seeding) and/or incubation time. In some examples, the meat culture bioreactor module and cells are rotated intermittently. In some examples, the meat culture bioreactor module and cells are rotated at a speed of between 0 to 30 rpm. In some examples, the meat culture bioreactor module and cells are rotated at a speed of about 12 rpm. In some examples, the meat culture bioreactor module and cells are rotated for 1 second to about 30 minutes every 10 seconds to 30 minutes. In some examples, the meat culture bioreactor module and cells are rotated for about 30 seconds every 5 minutes. That is to say that the meat culture bioreactor module is rotated for a period of 30 seconds and then remains stationary for 5 minutes before being rotated again. The meat culture bioreactor module may be rotated for at least 1 hour. For example, at least 1, 2, 3, 4 or 5 hours.

In some examples, the meat culture bioreactor module may be rotated continuously. The meat culture bioreactor module may be continuously rotated at a speed of between 0 and 30 rpm. In some examples, the meat culture bioreactor module is continuously rotated at a speed between 0 and 4 rpm. In some examples, the meat culture bioreactor module is continuously rotated for at least 3 hours. For example, about 3.5 to about 5 hours.

Rotation of the meat culture bioreactor module and cells may be used to exert a centrifugal force on the cells. As such, the centrifugal force may be more than 0.001 N. For example, the centrifugal force may be at least 0.001 N. In some examples, the centrifugal force may be between 0 and 50 N. The application of centrifugal force may help to move the cells through the meat culture bioreactor module and bring the cells into contact with the fibres. Without being bound by theory, this may help to improve the attachment efficiency of the cells to the fibres. Intermittent rotation of the meat culture bioreactor module may help introduce motive centrifugal forces to free-floating cells which may improve mixing. The increased mixing may help provide an even distribution of cells across fibre surfaces upon contact and attachment.

The force applied by rotation may be affected by the positioning of the meat culture bioreactor module in relation to the axis of rotation. For example, the meat culture bioreactor module may be positioned so as to have an offset between the mid-point of the axial centreline of the meat culture bioreactor module and the axis of rotation. The centreline of the meat culture bioreactor module refers to the plane running axially through the centre of meat culture bioreactor module longitudinally. The mid-point of the centre line refers to the distance halfway along the centreline. Providing an offset in combination with the rotation described above may help to mix the cells within the meat culture bioreactor module. Without being bound by theory, the mixing imparted by the offset and rotation may increase the surface area of the fibres that is contacted by the cells and may therefore improve the attachment efficiency of the cells to the fibres.

Maintaining the meat culture bioreactor module and/or rotation (intermittent or continuous) may help improve cell attachment and/or increase the number of viable of cells (i.e. viable cells attached to the hollow fibres). For example, increase the attachment efficiency in comparison to methods not including a maintaining step and/or rotation as described herein by at least 5%, 10%, 15%, 20%, 25%, 30%, 35%, 40%, 45%, 50%, 55%, 60%, 65%, 70%, 75%, 80%, 85%, 90%, 95%, 100% or more.

*Meat Culturing Method*

According to the present invention there is also provided a method of culturing meat, the method comprising:

providing the meat culturing system described above,

seeding the meat culture bioreactor system with muscle cells and/or muscle cell precursors, and

circulating a cell culture medium through the meat culture bioreactor module.

In certain embodiments, circulating the cell culture medium comprises pumping culture media through the meat culture bioreactor module (either through the internal lumina of the hollow fibres, or through the cavity). The cell culture media may be circulated at a rate of at least $10\mu$L/hour/fibre.

In particular, in some examples where the meat culture construct has hollow fibres, the cell culture media is circulated through the lumina of the hollow fibres. Nutrients (e.g., oxygen) in the cell culture media are transferred to cells growing on the outside of the hollow fibres by diffusion. In other examples where the meat culture construct has hollow fibres, the cell culture media is additionally or alternatively circulated through the cavity of the housing. In other examples where the meat culture construct has solid fibres, the cell culture media is circulated through the cavity of the housing.

In certain embodiments, the method also includes maintaining the meat culture construct (and cells) at a temperature of at least 15$\underline{o}$C and/or 5% $CO_2$.

In certain embodiments, the cell culture media is circulated through the meat culture bioreactor module for at least 3 hours.

In certain embodiments, a first culture media is first circulated through the meat culture bioreactor module, optionally wherein the first culture media is a proliferation medium.

In certain embodiments, the method further comprises circulating a second cell culture media through the meat culture bioreactor module, optionally wherein the second cell culture media is a differentiation medium; further optionally wherein the differentiation media is circulated through the meat culture bioreactor module for at least 3 hours.

In certain embodiments, the meat culture system comprises apparatus for monitoring metabolite concentration and/or oxygen concentration of the culture media.

In certain embodiments, the method further comprises harvesting the cultured meat (in particular the muscle cells) from the meat culture bioreactor module.

In certain embodiments, the harvested cultured meat is formed into a cultured meat product; or the one or more fibres are edible, and the harvested cells and the one or more fibres are formed into a cultured meat product.

In certain embodiments, the muscle cells and/or muscle cell precursors are derived from at least one comestible animal cell; optionally wherein the muscle cells or muscle cell precursors comprise one or more of fibroblasts, skeletal muscle cells, smooth muscle cells, and/or myoblasts; further optionally wherein the muscle cells ae derived from one or more of non-human embryonic stem cells and/or pluripotent stem cells.

The cell culture medium that may be used in the methods described herein may be any suitable cell culture medium. The cell culture medium may be selected depending on the type of cell cells being cultured. Examples of culture mediums that may be used include minimal essential medium (MEM, Sigma, St. Louis, Mo); Dulbecco's modified Eagle medium (DMEM, Sigma); Ham F10 medium (Sigma); Cell culture media (HyClone, Logan, Utah); RPMI-1640 culture media (Sigma); and chemical-defined (CD) culture media (which are formulated for individual cell types), such as CD-CHO culture media (Invitrogen, Carlsbad, Calif). The culture solution described above can be supplemented with auxiliary components or contents as needed. This includes any component of the appropriate concentration or amount required or desired.

The culture medium described above can be supplemented with auxiliary components or contents as needed. The culture medium may include one or more additives such as antibiotics, proteins, amino acids and/or sugars.

"Medium" and "cell culture medium" refer to a nutrient source used for growing or maintaining cells. As is understood by a person of skill in the art, the nutrient source may contain components required by the cell for growth and/or survival or may contain components that aid in cell growth and/or survival. Vitamins, essential or non-essential amino acids, trace elements, and surfactants (e.g., poloxamers) are examples of medium components. Any media provided herein may also be supplemented with any one or more of insulin, plant hydrolysates and animal hydrolysates.

"Culturing" a cell refers to contacting a cell with a cell culture medium under conditions suitable for the viability and/or growth and/or proliferation of the cell.

Perfusing the cell culture medium may include perfusing a first culture media and then subsequently perfusing one or more second cell culture mediums. The first cell culture medium may be a cell culture medium that is for proliferating cells and may be referred to as proliferation medium.

Proliferation medium may be a medium comprising a source of nutrients, such as vitamins, minerals, carbon and energy sources, and other beneficial compounds that facilitate the biochemical and physiological processes occurring during expansion or proliferation of cells. The proliferation medium may comprise one or more carbon sources, vitamins, amino acids, and inorganic nutrients. Representative carbon sources include monosaccharides, disaccharides, and/or starches. For example, the proliferation medium may contain one or more carbohydrates such as sucrose, fructose, maltose, galactose, mannose, and lactose. The proliferation medium may also comprise amino acids. Suitable amino acids may include amino acids commonly found incorporated into proteins as well as amino acids not commonly found incorporated into proteins, such as arginosuccinate, citrulline, canavanine, ornithine, and D-stereoisomers. The proliferation medium may also comprise growth promotion agents, such as serum, for example, foetal bovine serum (FBS). Examples of other growth promotion agents include growth factors (e.g. recombinant growth factors), bovine ocular fluid, sericin protein, earthworm heat inactivated coelomic fluid. In some examples, the proliferation media may be a serum free culture media and may optionally include additional components depending on the cells being cultured. For example, serum free culture medias include those commercially available from ThermoFisher, Lonza Bioscience and Merck. The proliferation medium may also comprise antibiotics.

For example, the proliferation medium may be Dulbecco's Modified Eagle's Medium (DMEM), which may include 10% (V/V) filter sterilised foetal bovine serum and 1% (V/V) penicillin/streptomycin solution.

In some examples, such as when the cells are derived from an insect, the proliferation media may be a media such as Gibco insect media available from ThermoFisher.

The cells may be maintained and cultured in proliferation medium for at least 3 hours. For example at least 3, 4, 5, 6, 7, 8, 9, 10, 11, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 125, 130, 135, 140, 145 or 150 hours. In some examples, the cells may be maintained in proliferation media continuously.

The cell culture media may be changed to a second cell culture medium. The second cell culture medium may be a differentiation medium. Differentiation medium refers to a medium designed to support the differentiation of cells, that is, supporting the process of a cell changing from one cell type to another. The differentiation medium may include one or more amino acids, antibiotics, vitamins, salts, minerals, or lipids. The differentiation medium may include at least one carbon source, such as a sugar. For example, glucose. The differentiation medium may include one or more proteins, amino acids or other additional acids. In some examples, the differentiation media includes one or more growth promotion agents, such as serum, for example, foetal bovine serum or horse serum. Examples of other growth promotion agents include growth factors (e.g. recombinant growth factors), bovine ocular fluid, sericin protein, earthworm heat inactivated coelomic fluid. In some examples, the differentiation media may be a serum free culture media and may optionally include additional components depending on the cells being cultured. For example, serum free culture medias include those commercially available from ThermoFisher, Lonza Bioscience and Merck. In some examples, the differentiation medium may be high-glucose DMEM (97%) supplemented with 2% horse serum and 1% penicillin/streptomycin solution.

The cells may be maintained and cultured in differentiation medium for at least 3 hours. For example at least 3, 4, 5, 6, 7, 8, 9, 10, 11, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70, 72, 74, 76, 78, 80, 82, 84, 86, 88, 90, 92, 94, 96, 98, 100, 102, 104, 106, 108, 110, 112, 114, 116, 118, 120, 125, 130, 135, 140, 145 or 150 hours.

In some examples, the culture media may be changed a number of times. For example, the cells may first be cultured in proliferation media; then, the culture media is switched to differentiation media. The differentiation media may then be switched to proliferation media. The proliferation media may be the same as before or may contain different components and/or concentrations of components to the initial proliferation media.

In some examples, the cells may be continuously cultured. That is to say that the cells are maintained in one or more culture mediums as described herein. The cells may be cultured for a first time period (e.g. at least 3 hours or more) in a first media, then for a second time period (e.g. at least 3 hours or more) in a second media and then cultured in a further media and continuously cultured with any number of changes of the culture media so that cells can be constantly proliferated and/or differentiated. In such examples, the cells may be harvested at predetermined cell densities or time points in order to avoid overcrowding, reduced viability and/or cell death.

The cell culturing conditions (e.g. for either or both proliferation or differentiation) may be selected depending on the cell type and/or cell source. In some examples, cells may be cultured (e.g. proliferated and/or differentiated) at a temperature of at least 15$\underline{o}$C. In some examples, the cells may be cultured at a temperature of at least 15$\underline{o}$C, 16°C, 17°C, 18°C, 19°C, 20°C, 21°C, 22°C, 23°C, 24°C, 25°C, 26°C, 27°C, 28°C, 29°C, 30°C, 31°C, 32°C, 33°C, 34°C, 35°C, 36°C, or 37°C.

For example, for culturing of insect cells, the cells may be cultured at a temperature of from 15$\underline{o}$C to 32 $\underline{o}$C.

For example, for culturing mammal cells, the cells may be cultured at a temperature of 37°C.

For example, for marine animal cells (such as crustaceans, fish or mollusc cells), cells may be cultured at a temperature of from 15$\underline{o}$C to 32 $\underline{o}$C. In some examples, marine animal cells may be cultured at a temperature between 15$\underline{o}$C to 30 $\underline{o}$C.

The cells may be cultured in defined atmospheric conditions. For example, the cells may be cultured in an atmosphere that has predetermined humidity and/or gas concentration. For example, cells may be cultured in an atmosphere including at least 5% $CO_2$.

In examples, the method may comprise a wash and/or sterilisation step preceding the seeding of cells to the meat culture construct.

In another aspect of the invention there is provided a comestible product comprising cultured meat (in particular muscle cells) obtained by a method as described above. The comestible product may be a cultured meat product.

A cultured meat product is a meat product comprising fragments of cultured meat along with flavouring and other additives. The cultured meat product may be mixed with other sources of protein, such as plant proteins.

In other examples, the bioreactor system, bioreactor module, and cell culture construct may be used to culture other types of cells, particularly other types of adherent cells.

The examples described above relate to a meat culturing system having a meat culture bioreactor module holding a meat culture construct. Such a system, module and construct are for culturing muscle cells to produce a cultured meat product.

## BRIEF DESCRIPTION OF THE FIGURES

Examples of the invention are described, by way of example only, with reference to accompanying drawings, in which:

**FIG. 1** shows a meat culturing system with a meat culture bioreactor module, with hollow fibres.

**FIG. 2** shows a cross-sectional view of a first example meat culture bioreactor module of FIG. 1.

**FIG. 3** shows a cross-sectional view of a second example meat culture bioreactor module of FIG. 1.

**FIG. 4** shows a second example meat culture bioreactor module of the meat culture bioreactor system, with solid fibres.

**FIG. 5** shows the meat culturing system with ancillary components.

**FIG. 6** shows a graph of the Fibre Distribution Parameter (Y axis) and fibre density (X axis) for different fibre diameters.

**FIG. 7** shows the graph of FIG. 6 with further constraints overlaid for hollow fibres.

**FIG. 8** shows the graph of FIG. 6 with further constraints overlaid for solid fibres.

## DETAILED DESCRIPTION

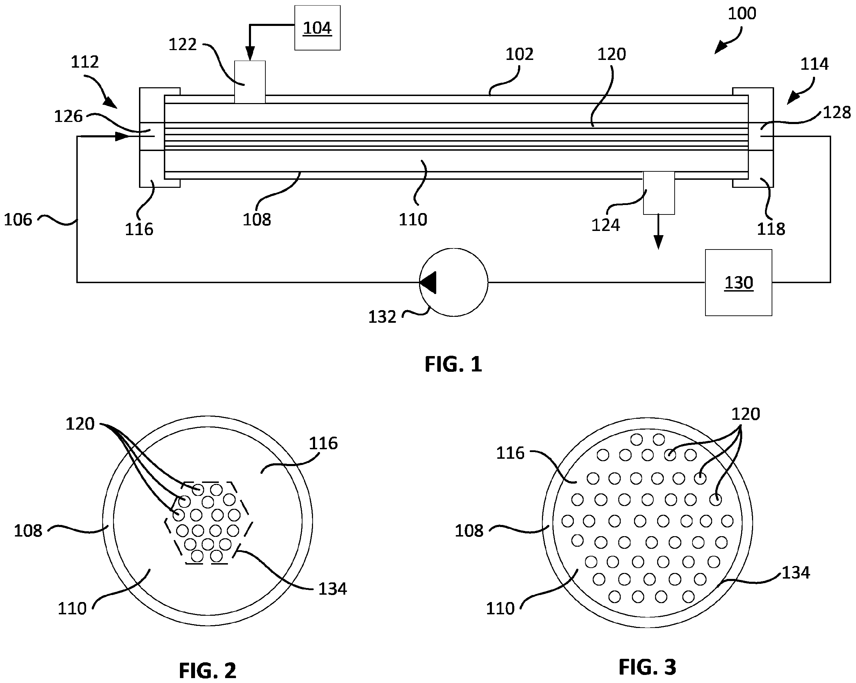

As shown in FIG. 1, the meat culturing system 100 includes a meat culture bioreactor module 102 connected with a cell supply system 104 and a cell culture media circulation system 106. In use, the cell supply system 104 is first used to introduce a cell suspension into the meat culture bioreactor module 102 in a seeding process in which the cells adhere (seed) to fibres within the meat culture bioreactor module 102. Subsequently, the cell culture media circulation system 106 is used to circulate a cell culture media through the meat culture bioreactor module 102 to facilitate cell culturing within the meat culture bioreactor module 102.

As illustrated in FIGS. 1, 2 and 3, the meat culture bioreactor module 102 includes a housing 108. The housing 108 is cylindrical and tubular, defining an internal cavity 110. The housing 108 and cavity 110 extend from a first end 112 to a second end 114. A first end support 116 is provided at the first end 112 and a second end support 118 is provided at the second end 114.

A plurality of hollow fibres 120 extend between the first end support 116 and the second end support 118. Ends of the hollow fibres 120 are embedded within the first and second end supports 116, 118 and the hollow fibres 120 extend longitudinally through the cavity 110.

An inlet 122 and outlet 124 are provided in a side wall of the housing 108 for providing a fluid connection with the cavity 110, and particularly the exterior surfaces of the hollow fibres 120 within the cavity 110. The inlet 122, and optionally the outlet 124, are in fluid communication with the cell supply system 104 for introducing a cell suspension to the cavity 110 during a cell seeding process.

The first and second end supports 116, 118 provide, respectively, an inlet 126 and an outlet 128 that are in fluid communication within the lumina of the hollow fibres 120. In particular, the external surfaces of the ends of the hollow fibres 120 are sealed within the first and second end supports 116, 118 and the lumina of the hollow fibres 120 are in fluid communication with the inlet 126 and outlet 128. The inlet 126 and outlet 128 are in fluid communication with the cell culture media circulation system 106 for circulation of a cell culture media through the hollow fibres 120 in a cell culturing process that occurs after cell seeding.

The cell culture media circulation system 106 includes a cell culture media source 130, which may be a storage container or the like, and a pump 132 for circulating the cell culture media through the hollow fibres 120.

The cell culture media circulation system 106 is operated during a cell culturing process, after cells have been seeded onto the external surfaces of the hollow fibres 120 within the housing 108. During this process nutrients (e.g., oxygen) are transferred from the cell culture media within the hollow fibres 120 to the cells on the external surface of the hollow fibres 120. This process may be a perfusion process.

As explained above, the ends of the hollow fibres 120 are embedded within the first end support 116. The arrangement of the hollow fibres 120 within the first end support 116 determines the spacings between the hollow fibres 120 and is important for the cell seeding process and the subsequent cell culturing process. In particular, as illustrated, in each example the hollow fibres 120 have a fibre distribution which is the arrangement of the hollow fibres 120 with respect to each other. In each example the fibre distribution is defined within an envelope 134. In the example of FIG. 2 the envelope 134 is smaller than the cavity 110 and so there is a space provided about the bundle of hollow fibres 120. In the example of FIG. 3 the envelope 134 is the same size as the cavity 110 and defined at the inner wall of the housing 108.

In the examples of FIG. 2 and FIG. 3 the hollow fibres 120 have a regular fibre distribution within the envelope 134. That is, the hollow fibres 120 are regularly distributed and evenly spaced from each other, defining a constant fibre density across the envelope 134. However, in other examples, the hollow fibres 120 may have a fibre distribution with varying fibre density across the envelope 134. For example, the fibre density may be greater towards a centre of the envelope 134 than towards an edge of the envelope 134 (i.e., the fibre spacing may be less towards a centre of the envelope 134 than towards an edge of the envelope 134).

In the example illustrated in FIG. 1 the hollow fibres 120 are embedded in the first end support 116 and in the second end support 118 at the second end 114 of the housing 108. However, in some examples the hollow fibres 120 may only be embedded within the first end support 116. In such examples the other ends of the hollow fibres 120 may instead be free within the cavity 110. In some examples both ends of the hollow fibres 120 may be embedded in the first end support 116, for example in different parts of the first end support 116 that form an inlet and an outlet.

The hollow fibres 120 may comprise a fibre distribution with a Fibre Distribution Parameter, FDP, of between 9 $cm^3$ and 24 $cm^3$.

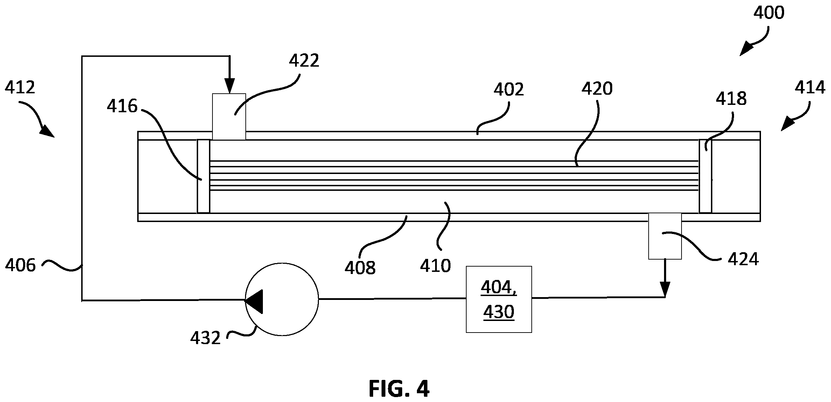

In the example of FIG. 4 the meat culturing system 400 includes a meat culture bioreactor module 402 connected with a cell supply system 404 and a cell culture media circulation system 406. In use, the cell supply system 404 is first used to provide cells to the meat culture bioreactor module 402 in a seeding process, and once seeded the cell culture media circulation system 406 is used to circulate a cell culture media through the meat culture bioreactor module 402 to facilitate cell culturing within the meat culture bioreactor module 402.

The meat culture bioreactor module 402 includes a housing 408. The housing 408 is cylindrical and tubular, defining an internal cavity 410. The housing 408 and cavity 410 extend from a first end 412 to a second end 414. A first end support 416 is provided at or near the first end 412 and a second end support 418 is provided at or near the second end 414.

In this example a plurality of solid fibres 420 extend between the first end support 416 and the second end support 418. Ends of the solid fibres 420 are embedded within the first and second end supports 416, 418 and the solid fibres 420 extend longitudinally through the cavity 410.

An inlet 422 and outlet 424 are provided in a side wall of the housing 408 for providing a fluid connection with the cavity 410, and particularly the exterior surfaces of the solid fibres 420 within the cavity 410. In contrast to the example of FIG. 1, in this example the inlet 422, and optionally the outlet 424, are selectively in fluid communication with the cell supply system 404 or the cell culture media circulation system 430. Accordingly, the inlet 422 and outlet 424 are used first to introduce a cell suspension for seeding the meat culture bioreactor module 402, and subsequently for circulation of a cell culture media by the cell culture media circulation system 430. A pump 432 may be provided as part of the cell supply system 404 and/or the cell culture media circulation system 430.

In examples, the solid fibres 420 may have a fibre distribution as described with reference to the hollow fibres 120 of FIG. 2 and FIG. 3. In particular, the solid fibres 420 may comprise a fibre distribution having a Fibre Distribution Parameter, FDP, of between 9 cm$^3$ and 24 cm$^3$.

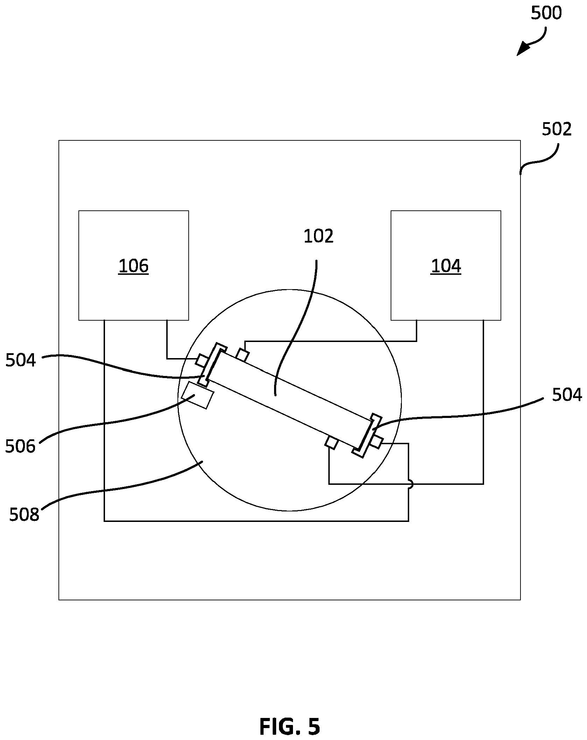

As shown in FIG. 5, during use the meat culture bioreactor module 102 may be mounted to a meat culturing system 500. The example of FIG. 5 shows the hollow fibre meat culture bioreactor module 102 of FIG. 1, but it will be appreciated that a similar system 500 can be used with the solid fibre meat culture bioreactor module 402 of FIG. 4, with the fluid connections adapted appropriately.,

As illustrated, the meat culture bioreactor module 102 is removably attached to the wider system 500 at mountings 504 that attach to each end of the meat culture bioreactor module 102. As shown, the mountings 504 hold the meat culture bioreactor module 102 at an incline from the horizontal, in particular at an incline of about 20 degrees from the horizontal. The mountings 504 also provide for fluid connections to the end supports 116, 118 to permit circulation of cell culture media as previously explained. The mountings 504 may be attached to a rotating base 508 that can be rotated (manually or automatically) to vary the incline angle of the meat culture bioreactor module 102.

The cell supply system 104 and the cell culture media circulation system 106 can be fluidly connected to the meat culture bioreactor module 102 by appropriate conduits and connectors.

An actuator, for example a motor 506, may be provided to rotate the meat culture bioreactor module 102 within the mountings 504. In particular, the motor 506 may rotate the meat culture bioreactor module 102 about its longitudinal axis.

During an initial seeding process the meat culture bioreactor module 102 is held at an inclined angle and rotated during introduction of the cell suspension. This helps the cell suspension to circulate through the meat culture bioreactor module 102 and evenly seed to the fibres therein. Subsequently, during a cell culturing process, the meat culture bioreactor module 102 may be maintained at an incline, or moved to a horizontal or vertical position.

*Tests*

The meat culture bioreactor modules are tested with a range of FDPs that demonstrate how the efficiency of seeding and attachment is improved by meat culture construct FDPs in the specified ranges.

To perform such a test the meat culture bioreactor module is disconnected from the meat culturing system. Cells are introduced via a side port (inlet) into the extra-capillary void volume (cavity). The meat culture bioreactor module ports are capped off, and the module held at an inclined angle and rotated for a defined time period, and then reconnected to the meat culturing system. After 1 day of culture, cells are harvested, counted and their viability assessed using a live/dead stain assay (acridine orange and 4',6-diamidino-2-phenylindole). A comparison of the total cells harvested compared to the total cells seeded gives a measure of the overall attachment efficiency.

To test cell culture progression as cells proliferate following seeding, meat culture bioreactor modules with a range of FDPs are continuously fed with culture media after seeding. Dissolved oxygen is measured at both inlet and outlet ports. It is desirable to maintain the dissolved oxygen level at the outlet consistent with the inlet level. As cells proliferate and their population increases, the flow rate of the medium supplied by the meat culture system is increased to maintain dissolved oxygen consistency between inlet and outlet ports. The flow rate can be related to the cell population to monitor cell proliferation in real time.

Meat culture bioreactor modules are run for between 1 and 25 days to proliferate cells, initially expanding in two-dimensions across the outer surface of the fibres before expanding into three-dimensional tissue and filling the extra-capillary void volume (spaces between the fibres). Furthermore, changing the growth medium to a formulation that triggers cell differentiation, for example, differentiating a muscle satellite cell into a myocyte, results in the stagnation of cell proliferation (no, or limited, further population increase) and the mass increase of cells through, in the example of muscle cells, an increase in proteins characteristic of muscle tissue, occurring over a further 1-25 day time period. During the period of cell mass increase, cells further fill the extra-capillary void volume. Harvesting of cells from the void space and quantifying via cell count quantification, live/dead cell viability assay, overall mass (yield) quantification, and confirmation of muscle protein presence, shows that greater yields are achieved by meat culture modules with the specified ranges of FDP.

A meat culture bioreactor module can be operated in a number of perfusion modes when operating with porous hollow fibres. Successful operation of the module is achieved by either flowing culture media from feed to retentate, with the permeate side port(s) closed. This results in mass transfer of nutrients solely by diffusion-controlled mass transfer into the extra-capillary space and diffusion of metabolites e.g. lactate or ammonia out of the extra-capillary space and back into the feed/retentate stream, and hence removal from the cell culture environment. A second mode of operation is achieved by closing the retentate flow and opening a/the permeate port(s). The pressure of the feed solution transports nutrients across the wall of a hollow fibre by convective mass transfer. Metabolites exit the meat culture module via the permeate port(s) along with spent growth media. The third is by controlling the amount of flow exiting either the retentate or permeate to achieve a required ratio of flow anywhere between the previous two operating modes. Operation of the module with varying proportions of convective and diffusive mass transfer enables control of the culture environment inside the meat culture bioreactor module to influence factors such as waste removal, stimulus of the cells through application of shear, or manipulation of nutrient distribution, all of which influence performance of the cell culturing process.

In order to measure the influence of operating mode, samples of media from the extra-capillary space can be taken from the permeate flow, the feed or the retentate. For example, lactate is an important metabolite to monitor across the duration of the cell culturing process, as when it reaches high levels it can reduce the performance of the cell culture process. In examples, it has been found that it can accumulate in the growth media present surrounding the cells on the extra-capillary side of hollow fibres when the fibres are at a low packing density, or when the pores are inadequately small. This is due to the diffusion of the lactate back into the feed-retentate stream being reduced due to the presence of higher than desirable mass transfer lengths encountered with low packing density, or that lactate is rejected by the porous hollow fibre to a greater degree than a hollow fibre with adequately sized pores. A further operating method to reduce lactate build up is to periodically increase the ratio of convective mass transport compared to diffusive mass transfer to purge the metabolite from the permeate port(s).

Unless defined otherwise herein, all technical and scientific terms used herein have the same meaning as commonly understood by one of ordinary skill in the art to which this invention pertains. For example, Singleton and Sainsbury, Dictionary of Microbiology and Molecular Biology, 2d Ed., John Wiley and Sons, NY (1994); and Hale and Marham, The Harper Collins Dictionary of Biology, Harper Perennial, NY (1991) provide those of skill in the art with a general dictionary of many of the terms used in the invention. Although any methods and materials similar or equivalent to those described herein find use in the practice of the present invention, the preferred methods and materials are described herein. Accordingly, the terms defined immediately below are more fully described by reference to the Specification as a whole. Also, as used herein, the singular terms "a", "an," and "the" include the plural reference unless the context clearly indicates otherwise. Unless otherwise indicated, nucleic acids are written left to right in 5' to 3' orientation; amino acid sequences are written left to right in amino to carboxy orientation, respectively. It is to be understood that this invention is not limited to the particular methodology, protocols, and reagents described, as these may vary, depending upon the context they are used by those of skill in the art.

## Claims

1.    A meat culture construct for a meat culture bioreactor module that is attachable to a meat culturing system, the meat culture bioreactor module having a housing adapted to receive the meat culture construct for culturing cells on the meat culture construct within the housing,

wherein the meat culture construct comprises a plurality of fibres and an end support, the plurality of fibres having external surfaces for cell adhesion and culturing, and having ends embedded within the end support and extending from the end support such they extend into the housing when the meat culture construct is received in the housing of the meat culture bioreactor module, and
wherein at a plane coincident with the end support the plurality of fibres are arranged in a fibre distribution defining an envelope, and wherein the fibre distribution comprises a Fibre Distribution Parameter, FDP, of between 9 cm$^3$ and 24 cm$^3$, the FDP being the product of the sum of the circumferences of the plurality of fibres within the envelope and the area within the envelope not occupied by the plurality of fibres.

2.    The meat culture construct of claim 1, wherein the FDP is between 10 cm$^3$ and 18 cm$^3$, for example between 11 cm$^3$ and 16 cm$^3$.

3.    The meat culture construct of claim 1 or claim 2, wherein the outer fibre diameter of the plurality of fibres is at least 0.3mm, for example at least 0.5mm.

4.    The meat culture construct of claim 1 or claim 2, wherein the outer fibre diameter of the plurality of fibres is between 0.3mm and 1 mm, for example between 0.4mm and 0.9mm, for example between 0.5mm and 0.8mm, for example between 0.5mm and 0.7mm, for example one of 0.3mm, 0.4mm, 0.5mm, 0.6mm, 0.7mm, 0.8mm, 0.9mm or 1mm.

5.    The meat culture construct of any of claims 1 to 4, wherein the plurality of fibres comprise a fibre density between about 50 fibres/cm$^2$ and about 330 fibres/cm$^2$.

6.    A meat culture construct for a meat culture bioreactor module that is attachable to a meat culturing system, the meat culture bioreactor module having a housing adapted to receive the meat culture construct for culturing cells on the meat culture construct within the housing,

wherein the meat culture construct comprises a plurality of fibres and an end support, the plurality of fibres having external surfaces for cell adhesion and culturing, and having ends embedded within the end support and extending from the end support such they extend into the housing when the meat culture construct is received in the housing of the meat culture bioreactor module, and

wherein at a plane coincident with the end support the plurality of fibres are arranged in a fibre distribution defining an envelope, and wherein the fibre distribution comprises a fibre density of between about 50 fibres/cm$^2$ and about 330 fibres/cm$^2$.

7. The meat culture construct of claim 6, wherein the outer fibre diameter of the plurality of fibres is at least 0.3mm, for example at least 0.5mm.

8. The meat culture construct of claim 6 or claim 7, wherein the outer fibre diameter of the plurality of fibres is between 0.3mm and 1 mm, for example between 0.4mm and 0.9mm, for example between 0.5mm and 0.8mm, for example between 0.5mm and 0.7mm, for example one of 0.3mm, 0.4mm, 0.5mm, 0.6mm, 0.7mm, 0.8mm, 0.9mm or 1mm.

9. The meat culture construct of any preceding claim, wherein a cross-sectional area of the envelope is smaller than the cross-sectional area of the end support.

10. The meat culture construct of any preceding claim, wherein at the end support the fibre density is substantially uniform across the envelope.

11. The meat culture construct of claims 1 to 19, wherein at the end support the fibre density varies across the envelope.

12. The meat culture construct of claim 11, wherein the fibre density at a centre of the fibre distribution is higher than a fibre density at an edge of the fibre distribution.

13. The meat culture construct of any preceding claim, further comprising a second end support, wherein opposite ends of the plurality of fibres are embedded in the second end support, and wherein the end support and second end support are receivable at opposite ends of the housing such that the plurality of fibres extend between the end support and the second end support within the housing.

14. The meat culture construct of any preceding claim, wherein the plurality of fibres are hollow fibres.

15. The meat culture construct of any of claims 1 to 14, wherein the plurality of fibres are solid fibres.

16. A meat culture bioreactor module comprising the meat culture construct of any of claims 1 to 15 and a housing having a cavity within which the meat culture construct is receivable such that the plurality of fibres extend into the cavity.

17. A meat culturing system comprising the meat culture bioreactor module of claim 16 and a base unit, the meat culture bioreactor module being detachable from the base unit.

18. The meat culturing system of claim 17, wherein the base unit is adapted to hold the meat culture bioreactor module such that the housing is inclined from a horizontal position.

19. The meat culturing system of claim 17 or claim 18, wherein the base unit comprises an actuator arranged to rotate the housing.

20. A method of seeding muscle cells and/or muscle cell precursors in the meat culture bioreactor module of claim 16, the method comprising:

    inclining the meat culture bioreactor module,
    introducing a cell suspension into the housing, and
    rotating the meat culture bioreactor module.

21. A method of culturing meat, the method comprising:

    providing the meat culturing system of any of claims 17 to 19,
    seeding the meat culture bioreactor system with muscle cells and/or muscle cell precursors, and
    circulating a cell culture medium through the meat culture bioreactor module.

**FIG. 1**

**FIG. 2**

**FIG. 3**

EP 4 588 999 A1

**FIG. 4**

EP 4 588 999 A1

**FIG. 5**

**FIG. 6**

**FIG. 7**

FIG. 8

## EUROPEAN SEARCH REPORT

Europäisches Patentamt
European Patent Office
Office européen des brevets

**Application Number**

EP 24 15 2625

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/038240 A2 (MERCK PATENT GMBH [DE]) 24 February 2022 (2022-02-24) * paragraphs [0009], [0014] - [0025], [0029], [0059], [0062], [0069] - [0073], [0076], [0077], [0086], [0087], [0100], [0104]; figures * ----- | 1-21 | INV. C12M3/00 C12M1/12 C12M3/06 |
| Y | CN 116 731 862 A (UNIV WUHAN TEXTILE) 12 September 2023 (2023-09-12) * page 1, paragraphs 2,6 * * page 2, paragraphs 5,7 * * page 3, paragraph 5 * ----- | 16-19 | |
| Y | US 2017/249873 A1 (HOGAN RICHARD J [US]) 31 August 2017 (2017-08-31) * paragraphs [0065] - [0069]; figures * ----- | 16-19 | |
| A | HANNA L TUOMISTO: "The eco-friendly burger", EMBO REPORTS, NATURE PUBLISHING GROUP, LONDON, GB, vol. 20, no. 1, 14 December 2018 (2018-12-14), page n/a, XP072242250, ISSN: 1469-221X, DOI: 10.15252/EMBR.201847395 * the whole document * ----- | 1,6,16, 20,21 | TECHNICAL FIELDS SEARCHED (IPC) C12M |
| A | WO 2023/152493 A1 (IVY FARM TECH LIMITED [GB]) 17 August 2023 (2023-08-17) * paragraphs [0001] - [0010], [0017] - [0022], [0031]; figure 1 * ----- | 1,6,16, 20,21 | |
| A | WO 2023/118872 A1 (CELLULAR AGRICULTURE LTD [GB]) 29 June 2023 (2023-06-29) * paragraphs [0051] - [0089]; figures * ----- | 1,6,16, 20,21 | |

-/--

The present search report has been drawn up for all claims

| Place of search | Date of completion of the search | Examiner |
|---|---|---|
| Berlin | 14 July 2024 | Böhm, Ingo |

EPO FORM 1503 03.82 (P04C01)

page 1 of 2

Europäisches
Patentamt

European
Patent Office

Office européen
des brevets

# EUROPEAN SEARCH REPORT

**Application Number**

EP 24 15 2625

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2023/284662 A1 (KAPLAN DAVID L [US] ET AL) 14 September 2023 (2023-09-14) * paragraphs [0033] - [0039]; figures 5,6 * | 1,6,16, 20,21 | |
| A | US 2010/210016 A1 (LEUTHAEUSER PAUL M [US] ET AL) 19 August 2010 (2010-08-19) * paragraphs [0003], [0029], [0053], [0055]; figures * | 16-19 | |

TECHNICAL FIELDS
SEARCHED      (IPC)

The present search report has been drawn up for all claims

| Place of search | Date of completion of the search | Examiner |
|---|---|---|
| Berlin | 14 July 2024 | Böhm, Ingo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.....................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 15 2625

This annex lists the patent family members relating to the patent documents cited in the above-mentioned European search report.
The members are as contained in the European Patent Office EDP file on
The European Patent Office is in no way liable for these particulars which are merely given for the purpose of information.

14-07-2024

| Patent document cited in search report | Publication date | Patent family member(s) | Publication date |
|---|---|---|---|
| WO 2022038240 A2 | 24-02-2022 | AU 2021328232 A1 | 02-03-2023 |
| | | CA 3190453 A1 | 24-02-2022 |
| | | CN 116348591 A | 27-06-2023 |
| | | EP 4199746 A2 | 28-06-2023 |
| | | IL 300593 A | 01-04-2023 |
| | | JP 2023539149 A | 13-09-2023 |
| | | KR 20230037658 A | 16-03-2023 |
| | | US 2024010983 A1 | 11-01-2024 |
| | | WO 2022038240 A2 | 24-02-2022 |
| CN 116731862 A | 12-09-2023 | NONE | |
| US 2017249873 A1 | 31-08-2017 | US 2014346765 A1 | 27-11-2014 |
| | | US 2017249873 A1 | 31-08-2017 |
| WO 2023152493 A1 | 17-08-2023 | NONE | |
| WO 2023118872 A1 | 29-06-2023 | GB 2614815 A | 19-07-2023 |
| | | WO 2023118872 A1 | 29-06-2023 |
| US 2023284662 A1 | 14-09-2023 | CN 116234453 A | 06-06-2023 |
| | | CN 116322810 A | 23-06-2023 |
| | | EP 4196187 A1 | 21-06-2023 |
| | | EP 4196569 A1 | 21-06-2023 |
| | | US 2023284662 A1 | 14-09-2023 |
| | | US 2023287317 A1 | 14-09-2023 |
| | | WO 2022036370 A1 | 17-02-2022 |
| | | WO 2022036371 A1 | 17-02-2022 |
| US 2010210016 A1 | 19-08-2010 | AU 2010216237 A1 | 18-08-2011 |
| | | EP 2398888 A1 | 28-12-2011 |
| | | JP 5643235 B2 | 17-12-2014 |
| | | JP 2012517828 A | 09-08-2012 |
| | | US 2010210016 A1 | 19-08-2010 |
| | | US 2013157366 A1 | 20-06-2013 |
| | | WO 2010096312 A1 | 26-08-2010 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LUETCHFORD, KIM A et al.** Next generation in vitro liver model design: Combining a permeable polystyrene membrane with a transdifferentiated cell line. *Journal of membrane science*, 2018, vol. 565, 425-438

- **SINGLETON ; SAINSBURY**. Dictionary of Microbiology and Molecular Biology. John Wiley and Sons, 1994
- **HALE ; MARHAM**. *The Harper Collins Dictionary of Biology*, 1991